# EUROPEAN PATENT APPLICATION

(11) **EP 2 260 864 A1**
(43) Date of publication of application: **15.12.2010**
(21) Application number: 09251529.5
(22) Date of filing: 10.06.2009
(51) Int. Cl.: A61K 39/395, A61K 38/00

(54) **Therapeutic applications**

(71) Applicant: University of Melbourne, Parkville VIC 3052 (AU); Howard Florey Institute, Carlton VIC 3053 (AU)
(72) Inventor: Turnley, Ann Maree, Melbourne, Victoria 3010 (AU); Butzkueven, Helmut, Parkville, Victoria 3010 (AU)
(74) Representative: Hallybone, Huw George

(57) **Abstract**

The specification relates to methods for treating a neurodegenerative disease such as multiple sclerosis by administering to a subject in need an EphA4 antagonist or an EphA4 ligand antagonist. The antagonist may be a soluble EphA4 or EphA4-binding ephrin or a functional variant thereof such as an EphA4-Fc or an ephrin A5-Fc. The antagonist may be an antibody or an antigen binding fragment, a nucleic acid, polypeptide, peptide, or organic molecule that binds to EphA4 or an EphA4-ligand or a nucleic acid encoding same and downregulates its activity. Combination therapies are also disclosed.

SEQUENCE LISTING

<110>  University of Melbourne
       Howard Florey Institute

<120>  Therapeutic applications

<130>  P053063EP

<160>  10

<170>  PatentIn version 3.5

<210>  1
<211>  20
<212>  PRT
<213>  mouse

<400>  1

Met Glu Val Gly Trp Arg Ser Pro Phe Ser Arg Val Val His Leu Tyr
1               5                   10                  15

Arg Asn Gly Lys
            20

<210>  2
<211>  777
<212>  PRT
<213>  mouse

<400>  2

Val Thr Gly Ser Arg Val Tyr Pro Ala Asn Glu Val Thr Leu Leu Asp
1               5                   10                  15

Ser Arg Ser Val Gln Gly Glu Leu Gly Trp Ile Ala Ser Pro Leu Glu
            20                  25                  30

Gly Gly Trp Glu Glu Val Ser Ile Met Asp Glu Lys Asn Thr Pro Ile
        35                  40                  45

Arg Thr Tyr Gln Val Cys Asn Val Met Glu Ala Ser Gln Asn Asn Trp
    50                  55                  60

Leu Arg Thr Asp Trp Ile Thr Arg Glu Gly Ala Gln Arg Val Tyr Ile
65                  70                  75                  80

Glu Ile Lys Phe Thr Leu Arg Asp Cys Asn Ser Leu Pro Gly Val Met
                85                  90                  95

Gly Thr Cys Lys Glu Thr Phe Asn Leu Tyr Tyr Tyr Glu Ser Asp Asn
            100                 105                 110

Asp Lys Glu Arg Phe Ile Arg Glu Ser Gln Phe Gly Lys Ile Asp Thr
        115                 120                 125

Ile Ala Ala Asp Glu Ser Phe Thr Gln Val Asp Ile Gly Asp Arg Ile
    130                 135                 140

Met Lys Leu Asn Thr Glu Ile Arg Asp Val Gly Pro Leu Ser Lys Lys
145                 150                 155                 160

Gly Phe Tyr Leu Ala Phe Gln Asp Val Gly Ala Cys Ile Ala Leu Val
                165                 170                 175

Ser Val Arg Val Phe Tyr Lys Lys Cys Pro Leu Thr Val Arg Asn Leu
            180                 185                 190

Ala Gln Phe Pro Asp Thr Ile Thr Gly Ala Asp Thr Ser Ser Leu Val
        195                 200                 205

Glu Val Arg Gly Ser Cys Val Asn Asn Ser Glu Glu Lys Asp Val Pro
    210                 215                 220

Lys Met Tyr Cys Gly Ala Asp Gly Glu Trp Leu Val Pro Ile Gly Asn
225                 230                 235                 240

Cys Leu Cys Asn Ala Gly His Glu Glu Gln Asn Gly Glu Cys Gln Ala
                245                 250                 255

Cys Lys Ile Gly Tyr Tyr Lys Ala Leu Ser Thr Asp Ala Ser Cys Ala
            260                 265                 270

Lys Cys Pro Pro His Ser Tyr Ser Val Trp Glu Gly Ala Thr Ser Cys
        275                 280                 285

Thr Cys Asp Arg Gly Phe Phe Arg Ala Asp Asn Asp Ala Ala Ser Met
    290                 295                 300

Pro Cys Thr Arg Pro Pro Ser Ala Pro Leu Asn Leu Ile Ser Asn Val
305                 310                 315                 320

Asn Glu Thr Ser Val Asn Leu Glu Trp Ser Ser Pro Gln Asn Thr Gly
                325                 330                 335

Gly Arg Gln Asp Ile Ser Tyr Asn Val Val Cys Lys Lys Cys Gly Ala
            340                 345                 350

Gly Asp Pro Ser Lys Cys Arg Pro Cys Gly Ser Gly Val His Tyr Thr
        355                 360                 365

Pro Gln Gln Asn Gly Leu Lys Thr Thr Arg Val Ser Ile Thr Asp Leu
    370                 375                 380

Leu Ala His Thr Asn Tyr Thr Phe Glu Ile Trp Ala Val Asn Gly Val
385                 390                 395                 400

Ser Lys Tyr Asn Pro Ser Pro Asp Gln Ser Val Ser Val Thr Val Thr
                405                 410                 415

Thr Asn Gln Ala Ala Pro Ser Ser Ile Ala Leu Val Gln Ala Lys Glu
            420                 425                 430

Val Thr Arg Tyr Ser Val Ala Leu Ala Trp Leu Glu Pro Asp Arg Pro
        435                 440                 445

Asn Gly Val Ile Leu Glu Tyr Glu Val Lys Tyr Tyr Glu Lys Asp Gln
    450                 455                 460

Asn Glu Arg Ser Tyr Arg Ile Val Arg Thr Ala Ala Arg Asn Thr Asp
465                 470                 475                 480

Ile Lys Gly Leu Asn Pro Leu Thr Ser Tyr Val Phe His Val Arg Ala
                485                 490                 495

Arg Thr Ala Ala Gly Tyr Gly Asp Phe Ser Glu Pro Leu Glu Val Thr
            500                 505                 510

Thr Asn Thr Val Pro Ser Arg Ile Ile Gly Asp Gly Ala Asn Ser Gly
        515                 520                 525

Ser Gly Gly Gly Gly Gly Gly Arg Ser Glu Asn Leu Tyr Phe Gln Gly
    530                 535                 540

Thr Glu Pro Lys Ser Cys Asp Lys Thr His Thr Cys Pro Pro Cys Pro
545                 550                 555                 560

Ala Pro Glu Leu Leu Gly Gly Pro Ser Val Phe Leu Phe Pro Pro Lys
                565                 570                 575

Pro Lys Asp Thr Leu Met Ile Ser Arg Thr Pro Glu Val Thr Cys Val
            580                 585                 590

Val Val Asp Val Ser His Glu Asp Pro Glu Val Lys Phe Asn Trp Tyr
        595                 600                 605

Val Asp Gly Val Glu Val His Asn Ala Lys Thr Lys Pro Arg Glu Glu
    610                 615                 620

Gln Tyr Asn Ser Thr Tyr Arg Val Val Ser Val Leu Thr Val Leu His
625                 630                 635                 640

Gln Asp Trp Leu Asn Gly Lys Glu Tyr Lys Cys Lys Val Ser Asn Lys
                645                 650                 655

Ala Leu Pro Ala Pro Ile Glu Lys Thr Ile Ser Lys Ala Lys Gly Gln
            660                 665                 670

Pro Arg Glu Pro Gln Val Tyr Thr Leu Pro Pro Ser Arg Asp Glu Leu
        675                 680                 685

Thr Lys Asn Gln Val Ser Leu Thr Cys Leu Val Lys Gly Phe Tyr Pro
    690                 695                 700

Ser Asp Ile Ala Val Glu Trp Glu Ser Asn Gly Gln Pro Glu Asn Asn
705                 710                 715                 720

Tyr Lys Ala Thr Pro Pro Val Leu Asp Ser Asp Gly Ser Phe Phe Leu
                725                 730                 735

Tyr Ser Lys Leu Thr Val Asp Lys Ser Arg Trp Gln Gln Gly Asn Val
            740                 745                 750

Phe Ser Cys Ser Val Met His Glu Ala Leu His Asn His Tyr Thr Gln
        755                 760                 765

Lys Ser Leu Ser Leu Ser Pro Gly Lys
    770                 775

<210>  3
<211>  750
<212>  PRT
<213>  mouse

<400>  3

Val Thr Gly Ser Arg Val Tyr Pro Ala Asn Glu Val Thr Leu Leu Asp
1               5                   10                  15

Ser Arg Ser Val Gln Gly Glu Leu Gly Trp Ile Ala Ser Pro Leu Glu
            20                  25                  30

Gly Gly Trp Glu Glu Val Ser Ile Met Asp Glu Lys Asn Thr Pro Ile
            35                  40                  45

Arg Thr Tyr Gln Val Cys Asn Val Met Glu Ala Ser Gln Asn Asn Trp
    50                  55                  60

Leu Arg Thr Asp Trp Ile Thr Arg Glu Gly Ala Gln Arg Val Tyr Ile
65                  70                  75                  80

Glu Ile Lys Phe Thr Leu Arg Asp Cys Asn Ser Leu Pro Gly Val Met
                85                  90                  95

Gly Thr Cys Lys Glu Thr Phe Asn Leu Tyr Tyr Tyr Glu Ser Asp Asn
            100                 105                 110

Asp Lys Glu Arg Phe Ile Arg Glu Ser Gln Phe Gly Lys Ile Asp Thr
        115                 120                 125

Ile Ala Ala Asp Glu Ser Phe Thr Gln Val Asp Ile Gly Asp Arg Ile
    130                 135                 140

Met Lys Leu Asn Thr Glu Ile Arg Asp Val Gly Pro Leu Ser Lys Lys
145                 150                 155                 160

Gly Phe Tyr Leu Ala Phe Gln Asp Val Gly Ala Cys Ile Ala Leu Val
                165                 170                 175

Ser Val Arg Val Phe Tyr Lys Lys Cys Pro Leu Thr Val Arg Asn Leu
            180                 185                 190

Ala Gln Phe Pro Asp Thr Ile Thr Gly Ala Asp Thr Ser Ser Leu Val
        195                 200                 205

Glu Val Arg Gly Ser Cys Val Asn Asn Ser Glu Glu Lys Asp Val Pro
    210                 215                 220

Lys Met Tyr Cys Gly Ala Asp Gly Glu Trp Leu Val Pro Ile Gly Asn
225                 230                 235                 240

Cys Leu Cys Asn Ala Gly His Glu Glu Gln Asn Gly Glu Cys Gln Ala
                245                 250                 255

Cys Lys Ile Gly Tyr Tyr Lys Ala Leu Ser Thr Asp Ala Ser Cys Ala
            260                 265                 270

Lys Cys Pro Pro His Ser Tyr Ser Val Trp Glu Gly Ala Thr Ser Cys
        275                 280                 285

Thr Cys Asp Arg Gly Phe Phe Arg Ala Asp Asn Asp Ala Ala Ser Met
    290                 295                 300

Pro Cys Thr Arg Pro Pro Ser Ala Pro Leu Asn Leu Ile Ser Asn Val
305                 310                 315                 320

Asn Glu Thr Ser Val Asn Leu Glu Trp Ser Ser Pro Gln Asn Thr Gly
                325                 330                 335

Gly Arg Gln Asp Ile Ser Tyr Asn Val Val Cys Lys Lys Cys Gly Ala
            340                 345                 350

Gly Asp Pro Ser Lys Cys Arg Pro Cys Gly Ser Gly Val His Tyr Thr
        355                 360                 365

Pro Gln Gln Asn Gly Leu Lys Thr Thr Arg Val Ser Ile Thr Asp Leu
    370                 375                 380

Leu Ala His Thr Asn Tyr Thr Phe Glu Ile Trp Ala Val Asn Gly Val
385                 390                 395                 400

Ser Lys Tyr Asn Pro Ser Pro Asp Gln Ser Val Ser Val Thr Val Thr
                405                 410                 415

Thr Asn Gln Ala Ala Pro Ser Ser Ile Ala Leu Val Gln Ala Lys Glu
            420                 425                 430

Val Thr Arg Tyr Ser Val Ala Leu Ala Trp Leu Glu Pro Asp Arg Pro
        435                 440                 445

Asn Gly Val Ile Leu Glu Tyr Glu Val Lys Tyr Tyr Glu Lys Asp Gln
    450                 455                 460

Asn Glu Arg Ser Tyr Arg Ile Val Arg Thr Ala Ala Arg Asn Thr Asp
465                 470                 475                 480

Ile Lys Gly Leu Asn Pro Leu Thr Ser Tyr Val Phe His Val Arg Ala
                485                 490                 495

Arg Thr Ala Ala Gly Tyr Gly Asp Phe Ser Glu Pro Leu Glu Val Thr
            500                 505                 510

Thr Asn Thr Val Pro Ser Arg Ile Ile Gly Asp Gly Ala Asn Ser Thr
        515                 520                 525

Gly Cys Lys Pro Cys Ile Cys Thr Val Pro Glu Val Ser Ser Val Phe
    530                 535                 540

Ile Phe Pro Pro Lys Pro Lys Asp Val Leu Thr Ile Thr Leu Thr Pro
545                 550                 555                 560

Lys Val Thr Cys Val Val Val Asp Ile Ser Lys Asp Asp Pro Glu Val
                565                 570                 575

Gln Phe Ser Trp Phe Val Asp Asp Val Glu Val His Thr Ala Gln Thr
            580                 585                 590

Gln Pro Arg Glu Glu Gln Phe Asn Ser Thr Phe Arg Ser Val Ser Glu
        595                 600                 605

Leu Pro Ile Met His Gln Asp Trp Leu Asn Gly Lys Glu Phe Lys Cys
    610                 615                 620

Arg Val Asn Ser Ala Ala Phe Pro Ala Pro Ile Glu Lys Thr Ile Ser
625                 630                 635                 640

Lys Thr Lys Gly Arg Pro Lys Ala Pro Gln Val Tyr Thr Ile Pro Pro
                645                 650                 655

Pro Lys Glu Gln Met Ala Lys Asp Lys Val Ser Leu Thr Cys Met Ile
            660                 665                 670

Thr Asp Phe Phe Pro Glu Asp Ile Thr Val Glu Trp Gln Trp Asn Gly
        675                 680                 685

Gln Pro Ala Glu Asn Tyr Lys Asn Thr Gln Pro Ile Met Asp Thr Asp
    690                 695                 700

Gly Ser Tyr Phe Val Tyr Ser Lys Leu Asn Val Gln Lys Ser Asn Trp
705                 710                 715                 720

Glu Ala Gly Asn Thr Phe Thr Cys Ser Val Leu His Glu Gly Leu His
                725                 730                 735

Asn His His Thr Glu Lys Ser Leu Ser His Ser Pro Gly Lys
            740                 745                 750

<210>  4

<211>  495
<212>  PRT
<213>  mouse

<400>  4

Ala Cys Lys Ile Gly Tyr Tyr Lys Ala Leu Ser Thr Asp Ala Ser Cys
1               5                   10                  15

Ala Lys Cys Pro Pro His Ser Tyr Ser Val Trp Glu Gly Ala Thr Ser
            20                  25                  30

Cys Thr Cys Asp Arg Gly Phe Phe Arg Ala Asp Asn Asp Ala Ala Ser
        35                  40                  45

Met Pro Cys Thr Arg Pro Pro Ser Ala Pro Leu Asn Leu Ile Ser Asn
    50                  55                  60

Val Asn Glu Thr Ser Val Asn Leu Glu Trp Ser Ser Pro Gln Asn Thr
65                  70                  75                  80

Gly Gly Arg Gln Asp Ile Ser Tyr Asn Val Val Cys Lys Lys Cys Gly
                85                  90                  95

Ala Gly Asp Pro Ser Lys Cys Arg Pro Cys Gly Ser Gly Val His Tyr
            100                 105                 110

Thr Pro Gln Gln Asn Gly Leu Lys Thr Thr Arg Val Ser Ile Thr Asp
        115                 120                 125

Leu Leu Ala His Thr Asn Tyr Thr Phe Glu Ile Trp Ala Val Asn Gly
    130                 135                 140

Val Ser Lys Tyr Asn Pro Ser Pro Asp Gln Ser Val Ser Val Thr Val
145                 150                 155                 160

Thr Thr Asn Gln Ala Ala Pro Ser Ser Ile Ala Leu Val Gln Ala Lys
                165                 170                 175

Glu Val Thr Arg Tyr Ser Val Ala Leu Ala Trp Leu Glu Pro Asp Arg
            180                 185                 190

Pro Asn Gly Val Ile Leu Glu Tyr Glu Val Lys Tyr Tyr Glu Lys Asp
        195                 200                 205

Gln Asn Glu Arg Ser Tyr Arg Ile Val Arg Thr Ala Ala Arg Asn Thr
    210                 215                 220

Asp Ile Lys Gly Leu Asn Pro Leu Thr Ser Tyr Val Phe His Val Arg
225                 230                 235                 240

Ala Arg Thr Ala Ala Gly Tyr Gly Asp Phe Ser Glu Pro Leu Glu Val
                245                 250                 255

Thr Thr Asn Thr Val Pro Ser Arg Ile Ile Gly Asp Gly Ala Asn Ser
            260                 265                 270

Thr Gly Cys Lys Pro Cys Ile Cys Thr Val Pro Glu Val Ser Ser Val
        275                 280                 285

Phe Ile Phe Pro Pro Lys Pro Lys Asp Val Leu Thr Ile Thr Leu Thr
    290                 295                 300

Pro Lys Val Thr Cys Val Val Val Asp Ile Ser Lys Asp Asp Pro Glu
305                 310                 315                 320

Val Gln Phe Ser Trp Phe Val Asp Asp Val Glu Val His Thr Ala Gln
                325                 330                 335

Thr Gln Pro Arg Glu Glu Gln Phe Asn Ser Thr Phe Arg Ser Val Ser
            340                 345                 350

Glu Leu Pro Ile Met His Gln Asp Trp Leu Asn Gly Lys Glu Phe Lys
        355                 360                 365

Cys Arg Val Asn Ser Ala Ala Phe Pro Ala Pro Ile Glu Lys Thr Ile
    370                 375                 380

Ser Lys Thr Lys Gly Arg Pro Lys Ala Pro Gln Val Tyr Thr Ile Pro
385                 390                 395                 400

Pro Pro Lys Glu Gln Met Ala Lys Asp Lys Val Ser Leu Thr Cys Met
                405                 410                 415

Ile Thr Asp Phe Phe Pro Glu Asp Ile Thr Val Glu Trp Gln Trp Asn
            420                 425                 430

Gly Gln Pro Ala Glu Asn Tyr Lys Asn Thr Gln Pro Ile Met Asp Thr
        435                 440                 445

Asp Gly Ser Tyr Phe Val Tyr Ser Lys Leu Asn Val Gln Lys Ser Asn
    450                 455                 460

Trp Glu Ala Gly Asn Thr Phe Thr Cys Ser Val Leu His Glu Gly Leu
465                 470                 475                 480

His Asn His His Thr Glu Lys Ser Leu Ser His Ser Pro Gly Lys
                485                 490                 495

<210>  5
<211>  533
<212>  PRT
<213>  mouse

<400>  5

Val Thr Gly Ser Arg Val Tyr Pro Ala Asn Glu Val Thr Leu Leu Asp
1               5                   10                  15

Ser Arg Ser Val Gln Gly Glu Leu Gly Trp Ile Ala Ser Pro Leu Glu
            20                  25                  30

Gly Gly Trp Glu Glu Val Ser Ile Met Asp Glu Lys Asn Thr Pro Ile
        35                  40                  45

Arg Thr Tyr Gln Val Cys Asn Val Met Glu Ala Ser Gln Asn Asn Trp
    50                  55                  60

Leu Arg Thr Asp Trp Ile Thr Arg Glu Gly Ala Gln Arg Val Tyr Ile
65                  70                  75                  80

Glu Ile Lys Phe Thr Leu Arg Asp Cys Asn Ser Leu Pro Gly Val Met
                85                  90                  95

Gly Thr Cys Lys Glu Thr Phe Asn Leu Tyr Tyr Tyr Glu Ser Asp Asn
            100                 105                 110

Asp Lys Glu Arg Phe Ile Arg Glu Ser Gln Phe Gly Lys Ile Asp Thr
        115                 120                 125

Ile Ala Ala Asp Glu Ser Phe Thr Gln Val Asp Ile Gly Asp Arg Ile
    130                 135                 140

Met Lys Leu Asn Thr Glu Ile Arg Asp Val Gly Pro Leu Ser Lys Lys
145                 150                 155                 160

Gly Phe Tyr Leu Ala Phe Gln Asp Val Gly Ala Cys Ile Ala Leu Val
                165                 170                 175

Ser Val Arg Val Phe Tyr Lys Lys Cys Pro Leu Thr Val Arg Asn Leu
            180                 185                 190

Ala Gln Phe Pro Asp Thr Ile Thr Gly Ala Asp Thr Ser Ser Leu Val
        195                 200                 205

Glu Val Arg Gly Ser Cys Val Asn Asn Ser Glu Glu Lys Asp Val Pro
    210                 215                 220

Lys Met Tyr Cys Gly Ala Asp Gly Glu Trp Leu Val Pro Ile Gly Asn
225                 230                 235                 240

Cys Leu Cys Asn Ala Gly His Glu Glu Gln Asn Gly Glu Cys Gln Ala
                245                 250                 255

Cys Lys Ile Gly Tyr Tyr Lys Ala Leu Ser Thr Asp Ala Ser Cys Ala
            260                 265                 270

Lys Cys Pro Pro His Ser Tyr Ser Val Trp Glu Gly Ala Thr Ser Cys
        275                 280                 285

Thr Cys Asp Arg Gly Phe Phe Arg Ala Asp Asn Asp Ala Ala Ser Met
    290                 295                 300

Pro Cys Thr Arg Pro Pro Ser Ala Pro Leu Asn Leu Ile Ser Asn Val
305                 310                 315                 320

Asn Glu Thr Ser Val Asn Leu Glu Trp Ser Ser Pro Gln Asn Thr Gly
                325                 330                 335

Gly Arg Gln Asp Ile Ser Tyr Asn Val Val Cys Lys Lys Cys Gly Ala
            340                 345                 350

Gly Asp Pro Ser Lys Cys Arg Pro Cys Gly Ser Gly Val His Tyr Thr
        355                 360                 365

Pro Gln Gln Asn Gly Leu Lys Thr Thr Arg Val Ser Ile Thr Asp Leu
    370                 375                 380

Leu Ala His Thr Asn Tyr Thr Phe Glu Ile Trp Ala Val Asn Gly Val
385                 390                 395                 400

Ser Lys Tyr Asn Pro Ser Pro Asp Gln Ser Val Ser Val Thr Val Thr
                405                 410                 415

Thr Asn Gln Ala Ala Pro Ser Ser Ile Ala Leu Val Gln Ala Lys Glu
            420                 425                 430

Val Thr Arg Tyr Ser Val Ala Leu Ala Trp Leu Glu Pro Asp Arg Pro
        435                 440                 445

Asn Gly Val Ile Leu Glu Tyr Glu Val Lys Tyr Tyr Glu Lys Asp Gln
    450                 455                 460

Asn Glu Arg Ser Tyr Arg Ile Val Arg Thr Ala Ala Arg Asn Thr Asp
465                 470                 475                 480

Ile Lys Gly Leu Asn Pro Leu Thr Ser Tyr Val Phe His Val Arg Ala
                485                 490                 495

Arg Thr Ala Ala Gly Tyr Gly Asp Phe Ser Glu Pro Leu Glu Val Thr
            500                 505                 510

Thr Asn Thr Val Pro Ser Arg Ile Ile Gly Asp Gly Ala Asn Ser His
        515                 520                 525

His His His His His
    530

<210>  6
<211>  278
<212>  PRT
<213>  human

<400>  6

Ala Cys Lys Ile Gly Tyr Tyr Lys Ala Leu Ser Thr Asp Ala Ser Cys
1               5                   10                  15

Ala Lys Cys Pro Pro His Ser Tyr Ser Val Trp Glu Gly Ala Thr Ser
            20                  25                  30

Cys Thr Cys Asp Arg Gly Phe Phe Arg Ala Asp Asn Asp Ala Ala Ser
        35                  40                  45

Met Pro Cys Thr Arg Pro Pro Ser Ala Pro Leu Asn Leu Ile Ser Asn
    50                  55                  60

Val Asn Glu Thr Ser Val Asn Leu Glu Trp Ser Ser Pro Gln Asn Thr
65                  70                  75                  80

Gly Gly Arg Gln Asp Ile Ser Tyr Asn Val Val Cys Lys Lys Cys Gly
                85                  90                  95

Ala Gly Asp Pro Ser Lys Cys Arg Pro Cys Gly Ser Gly Val His Tyr

            100                 105                 110

Thr Pro Gln Gln Asn Gly Leu Lys Thr Thr Arg Val Ser Ile Thr Asp
        115                 120                 125

Leu Leu Ala His Thr Asn Tyr Thr Phe Glu Ile Trp Ala Val Asn Gly
    130                 135                 140

Val Ser Lys Tyr Asn Pro Ser Pro Asp Gln Ser Val Ser Val Thr Val
145                 150                 155                 160

Thr Thr Asn Gln Ala Ala Pro Ser Ser Ile Ala Leu Val Gln Ala Lys
                165                 170                 175

Glu Val Thr Arg Tyr Ser Val Ala Leu Ala Trp Leu Glu Pro Asp Arg
            180                 185                 190

Pro Asn Gly Val Ile Leu Glu Tyr Glu Val Lys Tyr Tyr Glu Lys Asp
        195                 200                 205

Gln Asn Glu Arg Ser Tyr Arg Ile Val Arg Thr Ala Ala Arg Asn Thr
    210                 215                 220

Asp Ile Lys Gly Leu Asn Pro Leu Thr Ser Tyr Val Phe His Val Arg
225                 230                 235                 240

Ala Arg Thr Ala Ala Gly Tyr Gly Asp Phe Ser Glu Pro Leu Glu Val
                245                 250                 255

Thr Thr Asn Thr Val Pro Ser Arg Ile Ile Gly Asp Gly Ala Asn Ser
            260                 265                 270

His His His His His His
        275

<210> 7
<211> 756
<212> PRT
<213> human

<400> 7

Val Thr Gly Ser Arg Val Tyr Pro Ala Asn Glu Val Thr Leu Leu Asp
1               5                   10                  15

Ser Arg Ser Val Gln Gly Glu Leu Gly Trp Ile Ala Ser Pro Leu Glu
            20                  25                  30

Gly Gly Trp Glu Glu Val Ser Ile Met Asp Glu Lys Asn Thr Pro Ile
        35                  40                  45

Arg Thr Tyr Gln Val Cys Asn Val Met Glu Pro Ser Gln Asn Asn Trp
    50                  55                  60

Leu Arg Thr Asp Trp Ile Thr Arg Glu Gly Ala Gln Arg Val Tyr Ile
65                  70                  75                  80

Glu Ile Lys Phe Thr Leu Arg Asp Cys Asn Ser Leu Pro Gly Val Met
                85                  90                  95

Gly Thr Cys Lys Glu Thr Phe Asn Leu Tyr Tyr Tyr Glu Ser Asp Asn
            100                 105                 110

Asp Lys Glu Arg Phe Ile Arg Glu Asn Gln Phe Val Lys Ile Asp Thr
        115                 120                 125

Ile Ala Ala Asp Glu Ser Phe Thr Gln Val Asp Ile Gly Asp Arg Ile
    130                 135                 140

Met Lys Leu Asn Thr Glu Ile Arg Asp Val Gly Pro Leu Ser Lys Lys
145                 150                 155                 160

Gly Phe Tyr Leu Ala Phe Gln Asp Val Gly Ala Cys Ile Ala Leu Val
                165                 170                 175

Ser Val Arg Val Phe Tyr Lys Lys Cys Pro Leu Thr Val Arg Asn Leu
            180                 185                 190

Ala Gln Phe Pro Asp Thr Ile Thr Gly Ala Asp Thr Ser Ser Leu Val
        195                 200                 205

Glu Val Arg Gly Ser Cys Val Asn Asn Ser Glu Glu Lys Asp Val Pro
    210                 215                 220

Lys Met Tyr Cys Gly Ala Asp Gly Glu Trp Leu Val Pro Ile Gly Asn
225                 230                 235                 240

Cys Leu Cys Asn Ala Gly His Glu Glu Arg Ser Gly Glu Cys Gln Ala
                245                 250                 255

Cys Lys Ile Gly Tyr Tyr Lys Ala Leu Ser Thr Asp Ala Thr Cys Ala
            260                 265                 270

Lys Cys Pro Pro His Ser Tyr Ser Val Trp Glu Gly Ala Thr Ser Cys
        275                 280                 285

Thr Cys Asp Arg Gly Phe Phe Arg Ala Asp Asn Asp Ala Ala Ser Met
    290                 295                 300

Pro Cys Thr Arg Pro Pro Ser Ala Pro Leu Asn Leu Ile Ser Asn Val
305                 310                 315                 320

Asn Glu Thr Ser Val Asn Leu Glu Trp Ser Ser Pro Gln Asn Thr Gly
                325                 330                 335

Gly Arg Gln Asp Ile Ser Tyr Asn Val Val Cys Lys Lys Cys Gly Ala
            340                 345                 350

Gly Asp Pro Ser Lys Cys Arg Pro Cys Gly Ser Gly Val His Tyr Thr
        355                 360                 365

Pro Gln Gln Asn Gly Leu Lys Thr Thr Lys Val Ser Ile Thr Asp Leu
    370                 375                 380

Leu Ala His Thr Asn Tyr Thr Phe Glu Ile Trp Ala Val Asn Gly Val
385                 390                 395                 400

Ser Lys Tyr Asn Pro Asn Pro Asp Gln Ser Val Ser Val Thr Val Thr
                405                 410                 415

Thr Asn Gln Ala Ala Pro Ser Ser Ile Ala Leu Val Gln Ala Lys Glu
            420                 425                 430

Val Thr Arg Tyr Ser Val Ala Leu Ala Trp Leu Glu Pro Asp Arg Pro
        435                 440                 445

Asn Gly Val Ile Leu Glu Tyr Glu Val Lys Tyr Tyr Glu Lys Asp Gln
    450                 455                 460

Asn Glu Arg Ser Tyr Arg Ile Val Arg Thr Ala Ala Arg Asn Thr Asp
465                 470                 475                 480

Ile Lys Gly Leu Asn Pro Leu Thr Ser Tyr Val Phe His Val Arg Ala
                485                 490                 495

Arg Thr Ala Ala Gly Tyr Gly Asp Phe Ser Glu Pro Leu Glu Val Thr
            500                 505                 510

Thr Asn Thr Val Pro Ser Arg Ile Ile Gly Asp Gly Ala Asn Ser Glu

515                 520                 525

Ser Lys Tyr Gly Pro Pro Cys Pro Pro Cys Pro Ala Pro Glu Phe Leu
    530                 535                 540

Gly Gly Pro Ser Val Phe Leu Phe Pro Pro Lys Pro Lys Asp Thr Leu
545                 550                 555                 560

Met Ile Ser Arg Thr Pro Glu Val Thr Cys Val Val Val Asp Val Ser
                565                 570                 575

Gln Glu Asp Pro Glu Val Gln Phe Asn Trp Tyr Val Asp Gly Val Glu
            580                 585                 590

Val His Asn Ala Lys Thr Lys Pro Arg Glu Glu Gln Phe Asn Ser Thr
        595                 600                 605

Tyr Arg Val Val Ser Val Leu Thr Val Leu His Gln Asp Trp Leu Asn
    610                 615                 620

Gly Lys Glu Tyr Lys Cys Lys Val Ser Asn Lys Gly Leu Pro Ser Ser
625                 630                 635                 640

Ile Glu Lys Thr Ile Ser Lys Ala Lys Gly Gln Pro Arg Glu Pro Gln
                645                 650                 655

Val Tyr Thr Leu Pro Pro Ser Gln Glu Glu Met Thr Lys Asn Gln Val
            660                 665                 670

Ser Leu Thr Cys Leu Val Lys Gly Phe Tyr Pro Ser Asp Ile Ala Val
        675                 680                 685

Glu Trp Glu Ser Asn Gly Gln Pro Glu Asn Asn Tyr Lys Thr Thr Pro
    690                 695                 700

Pro Val Leu Asp Ser Asp Gly Ser Phe Phe Leu Tyr Ser Arg Leu Thr
705                 710                 715                 720

Val Asp Lys Ser Arg Trp Gln Glu Gly Asn Val Phe Ser Cys Ser Val
                725                 730                 735

Met His Glu Ala Leu His Asn His Tyr Thr Gln Lys Ser Leu Ser Leu
            740                 745                 750

Ser Leu Gly Lys
        755

<210> 8
<211> 396
<212> PRT
<213> human

<400> 8

<210>  9
<211>  421
<212>  PRT
<213>  human

<400>  9

Gln Asp Pro Gly Ser Lys Ala Val Ala Asp Arg Tyr Ala Val Tyr Trp
1               5                   10                  15

Asn Ser Ser Asn Pro Arg Phe Gln Arg Gly Asp Tyr His Ile Asp Val
            20                  25                  30

Cys Ile Asn Asp Tyr Leu Asp Val Phe Cys Pro His Tyr Glu Asp Ser
        35                  40                  45

Val Pro Glu Asp Lys Thr Glu Arg Tyr Val Leu Tyr Met Val Asn Phe
    50                  55                  60

Asp Gly Tyr Ser Ala Cys Asp His Thr Ser Lys Gly Phe Lys Arg Trp
65                  70                  75                  80

Glu Cys Asn Arg Pro His Ser Pro Asn Gly Pro Leu Lys Phe Ser Glu
                85                  90                  95

Lys Phe Gln Leu Phe Thr Pro Phe Ser Leu Gly Phe Glu Phe Arg Pro
            100                 105                 110

Gly Arg Glu Tyr Phe Tyr Ile Ser Ser Ala Ile Pro Asp Asn Gly Arg
        115                 120                 125

Arg Ser Cys Leu Lys Leu Lys Val Phe Val Arg Pro Thr Asn Ser Cys
    130                 135                 140

Met Lys Thr Ile Gly Val His Asp Arg Val Phe Asp Val Asn Asp Lys
145                 150                 155                 160

Val Glu Asn Ser Leu Glu Pro Ala Asp Asp Thr Val His Glu Ser Ala
                165                 170                 175

Glu Pro Ser Arg Gly Glu Asn Leu Tyr Phe Gln Gly Thr Glu Pro Lys
            180                 185                 190

Ser Cys Asp Lys Thr His Thr Cys Pro Pro Cys Pro Ala Pro Glu Leu
        195                 200                 205

Leu Gly Gly Pro Ser Val Phe Leu Phe Pro Pro Lys Pro Lys Asp Thr
    210                 215                 220

Leu Met Ile Ser Arg Thr Pro Glu Val Thr Cys Val Val Val Asp Val
225                 230                 235                 240

Ser His Glu Asp Pro Glu Val Lys Phe Asn Trp Tyr Val Asp Gly Val
                245                 250                 255

Glu Val His Asn Ala Lys Thr Lys Pro Arg Glu Glu Gln Tyr Asn Ser
            260                 265                 270

Thr Tyr Arg Val Val Ser Val Leu Thr Val Leu His Gln Asp Trp Leu
        275                 280                 285

Asn Gly Lys Glu Tyr Lys Cys Lys Val Ser Asn Lys Ala Leu Pro Ala
    290                 295                 300

Pro Ile Glu Lys Thr Ile Ser Lys Ala Lys Gly Gln Pro Arg Glu Pro
305                 310                 315                 320

Gln Val Tyr Thr Leu Pro Pro Ser Arg Asp Glu Leu Thr Lys Asn Gln
                325                 330                 335

Val Ser Leu Thr Cys Leu Val Lys Gly Phe Tyr Pro Ser Asp Ile Ala
            340                 345                 350

Val Glu Trp Glu Ser Asn Gly Gln Pro Glu Asn Asn Tyr Lys Ala Thr
        355                 360                 365

Pro Pro Val Leu Asp Ser Asp Gly Ser Phe Phe Leu Tyr Ser Lys Leu
    370                 375                 380

Thr Val Asp Lys Ser Arg Trp Gln Gln Gly Asn Val Phe Ser Cys Ser
385                 390                 395                 400

Val Met His Glu Ala Leu His Asn His Tyr Thr Gln Lys Ser Leu Ser
                405                 410                 415

Leu Ser Pro Gly Lys
            420

<210>  10
<211>  465
<212>  PRT
<213>  human

<400>  10

## Description

### FIELD

The specification relates to therapeutic neuroprotective and neuroregenerative molecules. In particular, the specification relates to therapeutic molecules that ameliorate the clinical progression of neurodegenerative diseases such as multiple sclerosis (MS) and variants of MS.

### BACKGROUND

Bibliographic details of references in the subject specification are also listed at the end of the specification.

Neural systems and in particular the central nervous system exhibits a limited capacity to regenerate after disease or injury, often with devastating consequences. Neural systems are an area of intense research and there appears to be some optimism in the art that at least some forms of acute spinal cord injury can now be repaired to some extent in mammalian models of spinal cord injury.

A large number of different molecules, cells and mechanisms have been implicated in the process of nerve regeneration. These were reviewed by Horner and Gage, Nature. 407(6807): 963-970, 2000. Of particular relevance here are the Eph and Ephrin receptorligand teams. There are four phylogenetic groups of Ephs and ephrins identified *inter alia* in man, mouse and chicken. Eph A receptors (of which there are currently ten members) bind to group A ephrin ligands (six members) which are GPI-linked membrane molecules, while the Eph B receptors (six members) bind to ephrin B ligands (three members) that are transmembrane molecules. Binding is promiscuous with most Eph A receptors binding to most ephrin A ligands and most Eph B receptors binding to most ephrin B ligands. Two exceptions are EphA4 which also binds to ephrin B2 and ephrin B3, and EphB2 which binds to ephrinA5.

The intracellular domain of transmembrane Eph receptors comprises a kinase domain linked to the membrane by a juxtamembrane domain. The carboxy side of the kinase domain comprises a SAM domain and a carboxy-terminal PDZ-binding domain. The extracellular portion comprises two fibronectin type III repeats, a cysteine rich region and an amino terminal ephrin binding domain. The ephrin B ligands have an extracellular Eph-binding domain, an intracellular portion comprising a short carboxy tail and a carboxy-terminal PDZ binding domain. The EphB-ephrinB complex has been crystallised and the structure is available for computational structure based identification of modulators of the interaction. The high affinity binding site between the receptor and the ligand comprises a fifteen amino acid long loop, the G-H loop in the Eph-binding domain that inserts into a cleft in the Eph receptor. Low affinity interaction sites appear *inter alia* to facilitate binding between two Eph-ephrin complexes. The interaction and clustering of receptors leads to activation of signaling pathways mediated by phosphorylation of residues in the cytoplasmic domain which promotes the kinase activity of the receptor. Bi-directional signalling occurs in which cell-based ephrin binding to cell-bound Eph receptors can induce signalling in either the ephrin or the Eph receptor. Antagonists of Eph/ephrin interaction are generally designed to block the high affinity or the low affinity interactions between members and hence receptor or ligand activation.

Ephs were first studied in the context of embryonic development where they were found to be critical axon guidance molecules for a number of neural systems. In these systems, complementary expression gradients of particular cell bound Eph/ephrin molecules effectively attract or repel growing axons in relation to their appropriate target. Eph/Ephrin interactions, including the actions of soluble forms are now thought to be involved in various ways in the development, maintenance and repair of number of different systems including neural, vascular and the immune system (see Wilkinson, Nat Rev Neurosci. 2(3): 155-164, 2001, Pasquale, Nat Rev Mol Cell Biol. 6(6): 462-75, 2005 and Horner and Gage, 2000 *(supra)).* Many studies have shown upregulation of Ephs and ephrins in neural tissue and reactive astrocytes following acute spinal injury or, hemisection of the spinal cord. Re-expression of these receptors in the adult has been postulated to be pivotal in modulating neural regeneration after acute injury (Goldshmit *et al.,* 2004 (*supra*), Goldshmit et al., Brain Res Brain Res Rev 52: 327-45, 2006). Blocking EphA4 on astrocytes by use of soluble ligands diminishes any directly inhibitory effects of EphA4 on axonal regrowth, as shown *in vitro* (Goldshmit *et al.,* 2004 (*supra*)).

Ephs and ephrin polypeptides can be used to activate or block activation of the Eph receptors, depending upon their stoichiometry. Clustering of ephrin-Fc ligands or membrane attachment of the ephrin ligands is required for Eph receptor activation, while when unclustered they block Eph receptor activation and the downstream cellular responses (Goldshmit *et al.,* 2004 (*supra*); Davis et al., Science 266: 816-9, 1994; Ohta et al.,. Mech Dev 64: 127-35, 1997; Stein et al., Genes Dev 12: 667-78, 1998).

A number of studies have shown that soluble Eph-Fc ligands are effective *in vivo,* specifically within the context of tumor growth inhibition (Brantley et al., Oncogene 21: 7011-26, 2002; Cheng et al., Neoplasia 5: 445-56, 2003, Dobrzanski et al., Cancer Res 64: 910-9, 2004).

Despite the significant amount of research that has been carried out into the role of Eph receptors in neural systems, it is not known whether there is any regulation or role of Eph/ephrin expression in CNS diseases, particularly MS and its model condition, experimental autoimmune encephalomyelitis (EAE).

MS is an inflammatory demyelinating neurodegenerative disease of the CNS. It is the most common cause of neurological disability in young adults and leads to permanent significant neurological disabilities over a number of years.

For many years MS was regarded primarily as an inflammatory demyelinating disease characterised by demyelination of axons in focal lesions within the CNS, with relative axonal sparing. Although axonal pathology was also noted (Barnes et al., Brain 114: 1271-80, 1991), it has only more recently become clear that there is substantial axonal damage and loss in MS lesions (Trapp el al., N Engl J Med 338: 278-85, 1998; Trapp et al., Curr Opin Neurol 12: 295-302, 1999). It is now widely believed that axonal loss is the cause of the irreversible permanent disability that is observed in the secondary or chronic, progressive phase of MS (Bjartmar et al., J Neurol Sci 206: 165-71, 2003). It is also apparent that axonal loss occurs in the earlier, relapsing-remitting phases of the disease and correlates with the extent of inflammation within the lesions, as indicated by histological analyses (Trapp *et al.,* 1998 (*supra*); Ferguson et al., Brain 120: 393-9, 1997; Kornek et al., Am J Pathol 157: 267-76, 2000) and imaging of human MS patient brains (De Stefano et al., Brain 121(Pt 8): 1469-77, 1998; Gonen et al., Neurology 54: .15-9, 2000). Furthermore, axonal loss can occur in the absence of myelin loss, in normal appearing white matter (Bjartmar et al., Neurology 57:1248-52, 2001), as well as in grey matter cortical lesions (Kidd et al., Brain 122 17-26, 1999; Peterson et al., Ann Neurol 50: 389-400, 2001). These and related findings, together with analysis of EAE models that correlated degree of axonal loss with clinical score (Wujek et al., J Neuropathol Exp Neurol 61: 23-32, 2002), have led to the hypothesis that the onset of the chronic, progressive phase of MS correlates with a threshold loss of axonal integrity (Bjartmar *et al,* 2003 (*supra*)). In the relapsing-remitting phase of MS, remyelination of demyelinated lesions occurs, however, there is little evidence to suggest that any axons that are damaged are able to regenerate. The MOG model of EAE is a useful tool in this regard as it produces substantial axonal loss (>40% in many regions) (Lo et al., J Neurophysiol 90: 3566-71, 2003; Wu et al., Neuroimage 37: 1138-47, 2007).

In MS and EAE one of the pathological hallmarks is the development of a glial scar in the demyelinated plaque, formed by activation of astrocytes (Raine et al., Lab Invest 31: 369-80, 1974; Smith et al., Brain Res 264: 241-53, 1983). Following CNS damage, astrocytes are activated, becoming hypertrophic and proliferative and increase expression of glial fibrillary acidic protein (GFAP). They also produce a range of soluble factors and extracellular matrix molecules. EphA4, together with EphA7, A6, A3 and A1 is also expressed on astrocytes in human MS tissue. However, it is not known whether this expression is merely a consequence of the injury or evidence of regeneration or both (Sobel, Brain Pathol. 15(1): 35-45, 2005).

Accordingly, an association of astrocytic gliosis with MS and EAE has long been accepted. However, it is not known whether this gliosis plays any role in the clinical progression and pathology of MS and EAE. Indeed, some studies have suggested that gliosis may have beneficial aspects in these conditions. For example, EAE in GFAP null mice resulted in a more severe clinical outcome (Liedtke et al., Am J Pathol 152: 251-9, 1998).

Investigations into the molecular mechanisms and cellular events characterising neurodegenerative disease have therefore lead to an increased understanding of the complex molecular/cellular pathways that are up regulated or down regulated and may be involved either in causing, or repairing neural damage. However, these investigations have, so far, permitted few conclusions concerning the way forward in terms of treatment. There is a need for new treatments for neurodegenerative diseases. Neuroprotective compositions are sought for a wide range of applications.

### SUMMARY

Historically, mouse models of experimental autoimmune encephalomyelitis (EAE) have been shown to reproduce specific features of histopathology and neuropathology of multiple sclerosis (MS) and to provide a useful indication of the therapeutic efficacy of putative modulatory agents in MS.

In accordance with the present invention, it has been determined surprisingly that the clinical course of experimental autoimmune encephalomyelitis (EAE) is less severe in EphA4 knockout mice and that axonal damage is decreased in EphA4 knockout mice with EAE compared to controls with EAE.

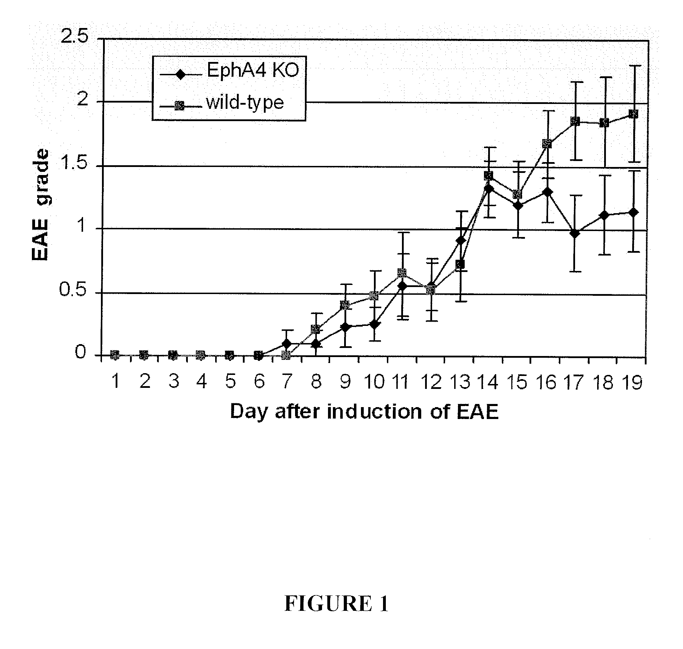

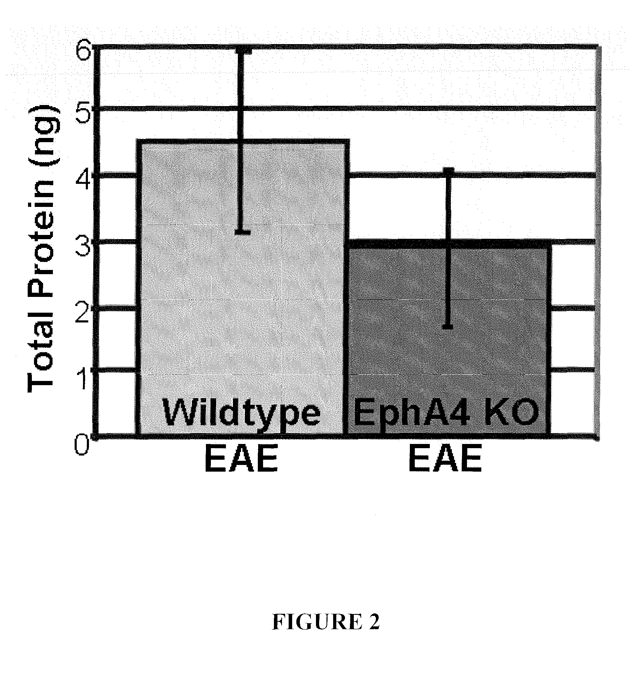

As shown in Figures 1 and 2, EphA4 deficient mice exhibited significantly less limb weakness and axonal damage compared to control EAE mice. Accordingly, it is proposed that the clinical course of neurodegenerative diseases (NDD), such as multiple sclerosis and variants of multiple sclerosis (MS), are ameliorated or otherwise attenuated by antagonising EphA4 or an EphA4-binding ligand (hereinafter "EphA4 ligand").

Each embodiment in this specification is to be applied *mutatis mutandis* to every other embodiment unless expressly stated otherwise.

In one embodiment, the present invention contemplates a method for treating a neurodegenerative disease (NDD) such as MS in a subject, the method comprising administering to the subject an EphA4 antagonist or an EphA4 ligand antagonist. In particular embodiments, the antagonist reduces clinical progression of the disease.

In a related embodiment, the invention contemplates an EphA4 antagonist or an EphA4 ligand antagonist for use in treating a neurodegenerative disease (NDD) such as MS in a subject. In particular embodiments, the antagonist reduces clinical progression of the disease.

In a similar embodiment, the invention broadly provides a use of an EphA4 antagonist or an EphA4 ligand antagonist in the manufacture of a medicament for treatment of a NDD such as MS in a subject. In particular embodiments, the antagonist reduces clinical progression of the disease. "Manufacture" includes selection and design of medicaments.

In another similar embodiment, the invention provides a pharmaceutical composition comprising an EphA4 antagonist or an EphA4 ligand antagonist for use in treating a NDD such as MS in a subject. In particular embodiments, the antagonist reduces clinical progression of the disease. For example, the antagonist may preserve the function or integrity of neural tissue or reduce the rate, occurrence or amount of neural damage that takes place in the subject.

In yet another embodiment, the invention contemplates a method for treating a NDD such as MS in a subject, the method comprising:
i) screening a biological sample from the subject for axon damage; and
ii) administering an EphA4 antagonist or an EphA4 ligand antagonist under conditions sufficient to preserve function or integrity of neural tissue or reduce the rate, occurrence or amount of neural damage that takes place in the subject.

In the above embodiments, the subject may be at risk of a NDD or exhibit clinical signs of a NDD.

In some embodiments, the NDD is MS or a variant of MS. Reference in this specification to "MS" includes MS and variants of MS.

In some embodiments, the antagonist is administered for a time and under conditions sufficient to reduce clinical progression of the disease. In some embodiments, the subject exhibits or is likely to exhibit less nerve damage or less progressive neurological dysfunction. In some embodiments, the neurological dysfunction is limb weakness. In some embodiments, an amount of antagonist is provided effective to reduce progressive neurological dysfunction.

In some embodiments, the antagonist preserves the function or integrity of neural tissue or reduces the rate, occurrence or amount of neural damage that takes place in a subject.

The term "damage" includes changes in the nerve (axon) that indicate loss of cellular integrity or loss of function. The term includes cell death, degeneration and fragmentation. In some embodiments the term extends to reduced nerve conductance or responsiveness or to demyelination. Neural damage may be assessed by any recognised procedure and as described herein.

In some embodiments, the antagonist preserves the function or integrity of neural tissue or reduces the rate, occurrence or amount of axon damage that takes place in a subject during the chronic progressive phases of a NDD such as MS. In some embodiments, administration is at or prior to the onset of clinical symptoms or prior to or at onset of relapse of clinical symptoms.

In some embodiments, the antagonist is administered to neural tissue or adjacent to neural tissue. In some embodiments, administration is local to the brain, spinal cord or optic nerve.

In some embodiments, the subject is a mammal such as a human.

In some embodiments, the antagonist is administered to provide between about 1 mg/kg to 100 mg/kg *per* day or every other day or *per* week systemically or a lower amount for local delivery.

EphA4 antagonists or EphA4 ligand antagonists are known in the art and are described in more detail in the Description and the Examples.

In some embodiments, the antagonist binds to EphA4 and down regulates its level or activity. In an exemplary embodiment, the antagonist is an ephrin including a soluble form or a functional variant or analog thereof that binds to EphA4 and down regulates its level or activity. Suitable ephrins are ephrin A ephrins, such as ephrin A1, A2, A3, A4, A5, A6, or ephrin B ephrins, such as ephrin B2 or B3. In some embodiments, the ephrin is A4, A5, B2 or B3. The ephrin may be monomeric, dimeric, tetrameric or multimeric. In some embodiments, the soluble ephrin is an Fc or His fusion protein. Fc fusions are particularly contemplated.

In other embodiments, the antagonist binds to an EphA4-binding ephrin molecule and inhibits its level or activity. In an exemplary embodiment, the antagonist is a EphA4 receptor such as a soluble EphA4 receptor polypeptide or a functional variant or analog thereof. In some embodiments, the EphA4 is monomeric, dimeric, tetrameric or multimeric. In some embodiments, the EphA4 receptor is an EphA4-Fc or His fusion protein. Fc fusions are particularly contemplated.

In other embodiments, the antagonist is an antisense or inhibitory RNA molecule.

In some embodiments, the antagonist is a protein, polypeptide or peptide such as, without limitation, a stapled peptide or a peptidomimetic. In other embodiments, the antagonist is a (typically small or medium sized) organic molecule such as, without limitation, a molecule derived from a library of pharmaceutically acceptable small organic molecules.

In some embodiments, the antagonist is an antibody or antigen-binding fragment of an antibody or aptamer that binds to EphA4 or an EphA4 ligand.

The present invention further provides for combination therapy such as targeting EphA4 signaling to decrease clinical signs of MS or other NDD, such as neural damage, and also providing an immunomodulatory or an immunosuppressive or an anti-inflammatory agent or a procedure for use in ameliorating immune or inflammatory components of the disease.

Thus, the invention contemplates a method for the treatment of a NDD such as MS comprising administering an EphA4 antagonist or an EphA4 ligand antagonist together with at least one other therapeutic agent or procedure. In a similar embodiment, the invention provides an EphA4 antagonist or an EphA4 ligand antagonist together with at least one other therapeutic agent or procedure for use in treating a NDD such as MS. In another related embodiment, the invention provides an EphA4 antagonist or an EphA4 ligand antagonist for use in treating a NDD such as MS, wherein the EphA4 antagonist or EphA4 ligand antagonist is to be administered together with at least one other therapeutic agent or procedure. Similarly, the invention provides at least one therapeutic agent or procedure for use in treating a NDD such as MS, wherein the therapeutic agent or procedure is to be administered together with an EphA4 antagonist or an EphA4 ligand antagonist. In some embodiments, the antagonist reduces clinical progression of the disease. Reference to "together" includes sequential or simultaneous administration.

In another aspect, the invention provides a use of an EphA4 antagonist or an EphA4 ligand antagonist in the manufacture of a composition for preserving function or integrity of neural tissue in a subject or for reducing the rate, occurrence or amount of neural damage in a subject. In some embodiments, the subject has or is at risk of having a condition associated with neural damage.

In a similar embodiment, methods are provided for preserving function or integrity of neural tissue or for reducing the rate, occurrence or amount of neural damage in a subject comprising administering an EphA4 antagonist or an EphA4 ligand antagonist to the subject. In some embodiments, the subject has or is at risk of having a condition associated with neural damage.

An EphA4 antagonist or an EphA4 ligand antagonist is similarly provided for use in preserving function or integrity of neural tissue or for reducing the rate, occurrence or amount of axonal damage in a subject. In some embodiments, the subject has or is at risk of having a condition associated with neural damage.

Similarly, a pharmaceutical composition is contemplated wherein the composition comprises an EphA4 antagonist or an EphA4 ligand antagonist for use in preserving function or integrity of neural tissue or for reducing the rate, occurrence or amount of axonal damage in a subject. In some embodiments, the subject has or is at risk of having a condition associated with neural damage.

Pharmaceutical compositions are formulated with a pharmaceutically acceptable carrier and/or diluent.

A medical kit is also provided comprising an EphA4 antagonist or an EphA4 ligand antagonist together with instructions for use in the treatment of a NDD such as MS.

The invention further provides a therapeutic protocol for treating a NDD such as MS in a subject, the protocol comprising in order, screening a biological sample from the subject for axon damage, administering an EphA4 antagonist or an EphA4 ligand antagonist for a time and under conditions proposed to be sufficient to reduce neural damage. In some embodiments, the subject is re-screened for axon damage after treatment. In some embodiments, alternatively or in addition, a sample from the subject is tested to determine the status of one or more conditions such as of autoimmunity, inflammation or demyelination. In some embodiments, the antagonist is administered prior to neural damage to prevent neural damage.

In another embodiment, the invention provides for screening of any one or more Eph or ephrin modulators for their ability to reduce the clinical progression of a NDD such as MS. In some embodiments, agents are tested for their ability to preserve function or integrity of neural tissue or to reducing the rate, occurrence or amount of axonal damage in a subject as illustrated in the Examples.

In other embodiments, screens for identifying agents for treatment of a NDD such as MS are provided comprising screening agents for their ability to modulate the level or activity of a polypeptide having the functional activity of EphA4 or an EphA4 ligand.

The above summary is not and should not be seen in any way as an exhaustive recitation of all embodiments of the present invention.

### BRIEF DESCRIPTION OF THE FIGURES

Some figures contain colour representations or entities. Coloured versions of the figures are available from the Patentee upon request or from an appropriate Patent Office. A fee may be imposed if obtained from a Patent Office.
**Figure 1** is a graphical representation showing that EphA4 knockout mice show a less severe clinical course of EAE. Disease onset and severity were similar between genotypes in the very early stages of the disease but followed a significantly different course thereafter. The maximum clinical grade reached by EphA4 knockout mice was lower than in controls (*n*=10 of each).
**Figure 2** is a graphical representation of ELISA data showing that EphA4 knockout mice with EAE show a trend for decreased levels of phospho-neurofilament-H indicative of less axonal damage in EAE than in wildtype mice with EAE.

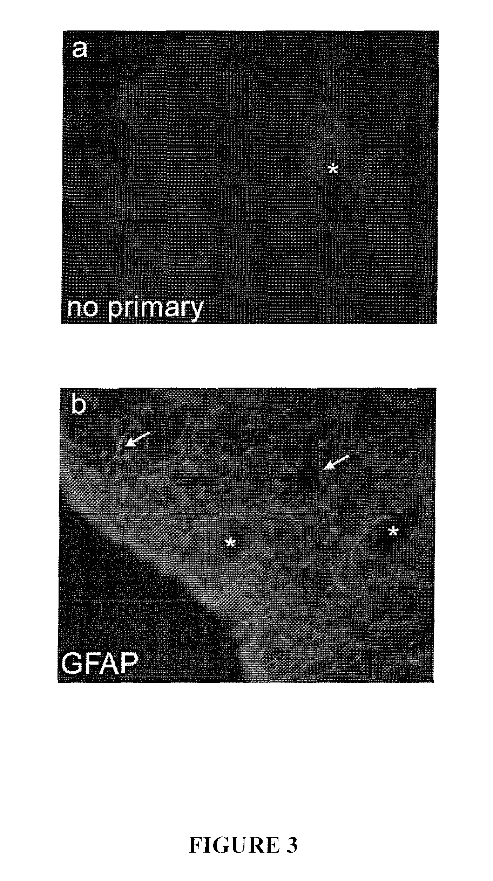

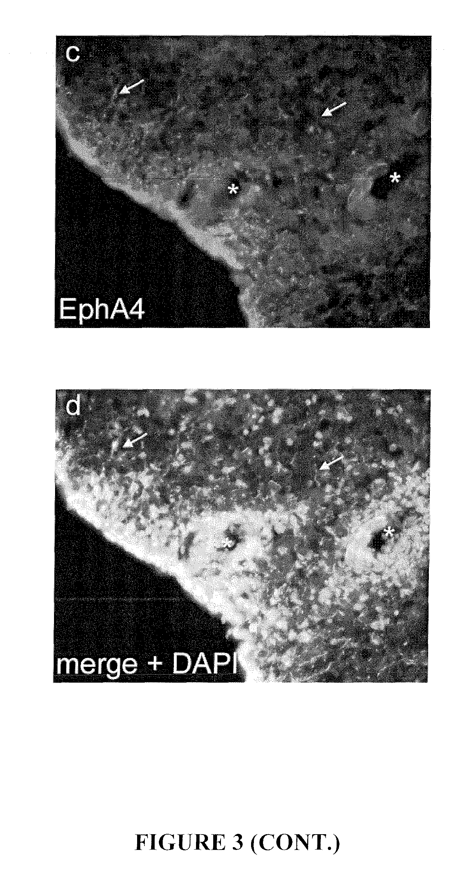

**Figure 3** **a through d** is a photographic representation showing expression of EphA4 around EAE lesion sites. Spinal cords from C57BL/6 mice with Grade 3 MOG-induced EAE were immunostained for GFAP and EphA4 expression. a) No overt background was present in the absence of primary EphA4 antibody; b) GFAP expressing astrocytes from these mice also expressed; c) EphA4 expression observable; d) merged b & c, plus DAPI to show nuclei. EphA4 is expressed on all GFAP positive astrocytes in EAE as indicated by red and green staining at site of arrow head in d). Two astrocytes are marked with arrows. Lesions are indicated by asterisks.
**Figure 4** is a photographic representation illustrating that EphrinA5-Fc enters the CNS at the injury site after IP injection. Mice were treated for 4 days with (A) PBS or (B) EphrinA5-Fc.
**Figure 5** is a photographic representation illustrating that monomeric EphrinA5-Fc blocks EphA4 phosphorylation on astrocytes. Astrocytes were treated *in vitro* with monomeric (blocking) or polymeric (activating) EphrinA5-Fc under basal conditions or with IFNγ.
Monomeric EphrinA5-Fc inhibited EphA4 phosphorylation under both conditions.
**Figure 6** is a diagrammatic representation of amino acid sequences of EphA4 and EphA4-binding ephrin Fc and His fusion proteins.

### BRIEF DESCRIPTION OF THE TABLES

**Table 1** provides a description of the SEQ ID NOs provided herein.
**Table 2** provides an amino acid sub-classification.
**Table 3** provides exemplary amino acid substitutions.
**Table 4** provides a list of non-natural amino acids contemplated in the present invention.
**Table 5** provides a list of abbreviations.

### DETAILED DISCUSSION OF EMBODIMENTS

### General

Abbreviations used herein are defined in Table 5.

Unless defined otherwise, all technical and scientific terms used herein have the same meanings as commonly understood by one of ordinary skill in the art to which this invention belongs. Any materials and methods similar or equivalent to those described herein can be used to practice or test the present invention. Practitioners are particularly directed to Sambrook et al., Molecular Cloning: A Laboratory Manual (2nd ed.). Cold Spring Harbour Laboratory, Cold Spring Harbour, NY, 1989, Coligan et al., Current Protocols In Protein Science, John Wiley & Sons, Inc., 1995-1997, in particular Chapters 1, 5 and 6. and Ausubel et al., Current Protocols in Molecular Biology, Supplement 47, John Wiley & Sons, New York, 1999 for definitions and terms of the art and other methods known to the person skilled in the art.

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

Nucleotide and amino acid sequences are referred to by sequence identifier numbers (SEQ ID NO:). The SEQ ID NOs: correspond numerically to the sequence identifiers <400>1, <400>2, etc. A summary of sequence identifiers is provided in Table 1. A sequence listing is provided after the claims.

The terms "polypeptide" "protein" and "peptide" are used interchangeably herein.

As used herein the singular forms "a", "an" and "the" include plural aspects unless the context clearly dictates otherwise. Thus, for example, reference to "a cell" includes a single cell, as well as two or more cells; reference to "an agent" includes one agent, as well as two or more agents; and so forth.

The terms "genetic material", "genetic forms", "nucleic acids", "nucleotide" and "polynucleotide" include RNA, cDNA, genomic DNA, synthetic forms and mixed polymers, both sense and antisense strands, and may be chemically or biochemically modified or may contain non-natural or derivatized nucleotide bases, as will be readily appreciated by those skilled in the art. Such modifications include, for example, labels, methylation, substitution of one or more of the naturally occurring nucleotides with an analog (such as the morpholine ring), internucleotide modifications such as uncharged linkages (e.g. methyl phosphonates, phosphotriesters, phosphoamidates, carbamates, etc.), charged linkages (e.g. phosphorothioates, phosphorodithioates, etc.), pendent moieties (e.g. polypeptides), intercalators (e.g. acridine, psoralen, etc.), chelators, alkylators and modified linkages (e.g. α-anomeric nucleic acids, etc.). Also included are synthetic molecules that mimic polynucleotides in their ability to bind to a designated sequence *via* hydrogen binding and other chemical interactions. Such molecules are known in the art and include, for example, those in which peptide linkages substitute for phosphate linkages in the backbone of the molecule.

The term "gene" is used in its broadest sense and includes cDNA corresponding to the exons of a gene. Reference herein to a "gene" is also taken to include: a classical genomic gene consisting of transcriptional and/or translational regulatory sequences and/or a coding region and/or non-translated sequences (i.e. introns, 5'- and 3'- untranslated sequences); or mRNA or cDNA corresponding to the coding regions (i.e. exons) and 5'- and 3'-untranslated sequences of the gene.

By "isolated" is meant material that is substantially or essentially free from components that normally accompany it in its native state. For example, an "isolated polynucleotide", as used herein, refers to a polynucleotide, isolated from the sequences which flank it in a naturally-occurring state, e.g., a DNA fragment which has been removed from the sequences that are normally adjacent to the fragment. Alternatively, an "isolated peptide" or an "isolated polypeptide" and the like, as used herein, refer to *in vitro* isolation and/or purification of a peptide or polypeptide molecule from its natural cellular environment, and from association with other components of the cell. Without limitation, an isolated composition, complex, polynucleotide, peptide, or polypeptide can refer to a native sequence that is isolated by purification or to a sequence that is produced by recombinant or synthetic means.

The citation of any reference herein should not be construed as an admission that such reference is available as "Prior Art" to the instant application.

The subject invention is not limited to particular screening procedures for agents. Specific formulations of agents and various medical methodologies may vary. The terminology used herein is for the purpose of describing particular embodiments only and is not intended to be limiting.

Reference to "treatment" or "treat" includes prophylactic, preventative measures as well as therapeutic steps where the object is to at least reduce the severity of clinical signs of neurodegenerative disease or to delay or reduce clinical progression of the disease. Without wishing to be bound by theory, the experimental results described herein indicate that reduction in EphA4 function preserves the function or integrity of neural tissue or provides a reduced rate, occurrence or amount of axonal damage contributing to better clinical outcome.

### Embodiments

In one embodiment, the present invention contemplates a method for treating a neurodegenerative disease (NDD) such as MS in a subject, the method comprising administering to the subject an EphA4 antagonist or an EphA4 ligand antagonist.

The term "antagonist" is used in a broad context to refer to an agent, active, modulator, medicament, compound, pharmacologically active agent, drug, molecule, etc that down regulates the level or activity of a target molecule. In this context, "activity" includes one or more biological functions of the target molecule. In some embodiments, the target is an Eph or ephrin polypeptide molecule. As known in the art, the activities of these molecules include *inter alia* intracellular signaling activities, enzymatic activities and binding activities that create their functional abilities. Signaling activity includes intracellular transduction of signals to effect cellular responses mediated by, for example, expression, phosphorylation or binding or conformational changes of intracellular factors; enzymatic activity includes kinase or other catalytic activity; and binding activity includes low medium or high affinity binding to complementary Ephs, ephrins or other molecules that form complexes or bind to Ephs or ephrins. As known in the art, in some embodiments, binding is inhibitory while in other embodiments, binding results in activation and receptor/ligand signaling. These functions are readily assessed and quantified. The activity of functional domains such as the juxtamembrane domain, PDZ-domain or SAM-domain, GPI anchors, fibronectin repeats and cysteine rich domains are also influential in modulating the functional activities of Ephs and ephrins. Methods for monitoring the level or activity of Eph/ephins or other ligands are known in the art. For example, some antagonists may be identified using EphA4 receptor binding assays, *e.g.* as described in Davis *et al.,* 1994 (*supra*), Murai et al., Mol Cell Neurosci. 24(4): 1000-1011, 2004 or PubChem Bioassay AID No. 689 (*Colorimetric assay for HTS discovery of chemical inhibitors of EphA4 receptor antagonists*). Suitable EphA4 antagonists and EphA4 ligand antagonists may inhibit the binding of EphA4 ligands to EphA4. Similarly, other antagonists may be identified using EphA4 receptor phosphorylation assays, *e.g.* as described in Davis *et al.,* 1994 (*supra*). Suitable EphA4 antagonists and EphA4 ligand antagonists may inhibit the phosphorylation of EphA4 when it is exposed to an EphA4 ligand. Functional assays may also be used to identify some antagonists, e.g. the neural crest migration assay in Murai *et al.,* 2004 (*supra*). Suitable EphA4 antagonists and EphA4 ligand antagonists may inhibit the function of EphA4 *in vitro* or *in vivo. In silico* methods may also be used to identify some antagonists. For example, the crystal structure of the high affinity ephrin-binding channel of the EphA4 receptor is known from Qin et al., J Biol Chem. 283(43): 29473-29484, 2008. This structure may be used for the rational design of suitable EphA4 antagonists and EphA4 ligand antagonists.

In some embodiments, the level or activity of a target polypeptide may be decreased by reducing the level of transcription or translation, such as by inhibiting promoter or enhancer activity, by methylation, or by the use of gene silencing such as co-suppression, antisense or inhibitory RNA strategies now routine in the art. Thus, the invention encompasses the use of nucleic acid molecule or vectors comprising nucleic acid molecules if required to indirectly modulate the level of EphA4 or an EphA4 ligand by, for example, gene therapy, or inhibiting or promoting the function of a gene, or using gene silencing constructs or antisense or inhibitory RNA oligonucleotides known to those of skill in the art. Methods for modulating and monitoring gene function including transcription or translation are known in the art. For example, a method for modulating and monitoring the expression of EphA4 is disclosed in Fu et al., Nature Neuroscience 10: 67-76, 2007.

The term "subject" as used herein refers to a warm blooded animal, in particular a mammal and more particularly a primate including a lower primate and even more particularly, a human who can benefit from the medical uses of the present invention. A subject regardless of whether a human or non-human animal or embryo may be referred to as an individual, subject, animal or patient. In some embodiments, the subject is a mammal such as a human. In some embodiments, the subject is a subject in need of treatment.

Reference to modulating or reducing the level or activity of a particular "Eph", "Eph-binding ligand" or "Ephrin" includes reference to all polypeptide forms of these molecules such as homologs from other species, or naturally occurring forms. Reference to these molecules as antagonists includes homologs, and naturally occurring, isolated, synthetic, or recombinant forms, analogs, and parts thereof such as functional fragments or domains that retain activity and functionally variant forms.

In some embodiments the subject is at risk of a NDD or exhibits one or more clinical signs of a NDD. A subject may, for example, have a genetically, clinically or physiologically demonstrable risk or likelihood of developing a NDD. For example, early clinical signs of MS include optic neuritis in subjects between 15 and 50 years of age.

Neurodegenerative diseases (NDD) include those of CNS and/or PNS tissue and include: peripheral neuropathies, motor neuron disease, amylotrophic lateral sclerosis (ALS, Lou Gehrig's disease), Bell's palsy, Alzheimer's disease, Parkinson's disease, epilepsy, multiple sclerosis, Huntington's chorea, Down's Syndrome, hypoxic-ischemic encephalopathy, incidental Lewy bodies, amyloid angiopathy, traumatic myelomalacia, and Meniere's disease. Peripheral neuropathy is a neurodegenerative disease that affects the peripheral nerves, most often manifested as one or a combination of motor, sensory, sensorimotor, or autonomic dysfunction. Peripheral neuropathies may, for example, be genetically acquired, can result from a systemic disease, or can be induced by a toxic agent, such as a neurotoxic drug, for example an antineoplastic agent, or industrial or environmental pollutant. Peripheral sensory neuropathy is characterised by the degeneration of peripheral sensory neurons, which may be idiopathic, may occur, for example, as a consequence of diabetes (diabetic neuropathy), cytostatic drug therapy in cancer, alcoholism, acquired immunodeficiency syndrome (AIDS), or genetic predisposition. Genetically acquired peripheral neuropathies include, for example, Refsum's disease, Krabbe's disease, Metachromatic leukodystrophy, Fabry's disease, Dejerine-Sottas syndrome, Abetalipoproteinemia, and Charcot-Marie-Tooth (CMT) disease (also known as Proneal Muscular Atrophy or hereditary motor sensory neuropathy (HMSN)). Peripheral neuropathy usually affects sensory and motor nerves together so as to cause a mixed sensory and motor neuropathy, but pure sensory and pure motor neuropathy are also known. In some embodiments, symptoms of NDD develop over a course of several months or years where they are referred to as chronic or progressive.

In some embodiments, the neurodegenerative disease is associated with one or more of demyelination, inflammation and autoimmunity. Examples of diseases that are associated with demyelination, inflammation and autoimmunity are multiple sclerosis and its variants or Guillain-Barre syndrome (GBS) and its variants..

In some embodiments, the neurodegenerative disease is multiple sclerosis or a variant of MS. Variants of MS include Marburg's variant, Schilder's disease, concentric sclerosis of Balo and Devic's disease. In an illustrative embodiment, the disease (condition) is multiple sclerosis.

In some embodiments, the antagonist is administered for a time and under conditions sufficient to reduce clinical progression of the disease. In some embodiments, the subject exhibits or is likely to exhibit less nerve damage or less progressive neurological dysfunction. In some embodiments, the neurological dysfunction is limb weakness. In some embodiments, an amount of antagonist is provided effective to reduce progressive neurological dysfunction.

In some embodiments, the antagonist preserves the function or integrity of neural tissue or reduces the rate, occurrence or amount of neural damage that takes place in a subject.

In some embodiments, an antagonist is provided in an "effective amount" sufficient to ameliorate or delay progress of clinical signs of a NDD such as MS.

By "effective amount," in the context of treating a NDD is meant the administration of that amount of active to a subject, either in a single dose or as part of a series or slow release system, that has been shown to be effective in showing a therapeutic effect in some subjects. The effective amount will vary depending upon the health and physical condition of the subject the formulation of the composition used, the assessment of the medical situation, and other relevant factors. It is expected that the amount will fall in a relatively broad range that can be determined through routine trials.

The pharmaceutical composition comprising the subject antagonists is contemplated to exhibit therapeutic activity when administered in an amount which depends on the particular case. The variation depends, for example, on the human or animal and the agent chosen. Antagonists are typically agents that are biologically available at concentrations sufficient to be effective. A broad range of doses may be applicable. Considering a subject, for example, from about 0.1 mg to 0.9mg (i.e., including 0.1 mg, 0.2 mg, 0.3 mg, 0.4 mg, 0.5 mg, 0.6 mg, 0.7 mg, 0.8 mg. and 0.9 mg), from about 15 mg to 35 mg, about 1 mg to 30 mg or from 5 to 50 mg, or from 10 mg to 100 mg of agent may be administered *per* kilogram of body weight *per* day or *per* every other day or *per* week or *per* month. Therapeutic antibodies are typically administered at a dosage of about 1 to 20 mg/kg however dosages above or below this amount are contemplated in the ranges set out above. Dosage regimes may be adjusted to provide the optimum therapeutic response. For example, several divided doses may be administered daily, weekly, monthly or other suitable time intervals or the dose may be proportionally reduced as indicated by the exigencies of the situation.

In some embodiments, the antagonist is administered to provide about 1 mg to 100 mg/kg *per* day or week systemically or a lower amount for local delivery.

Administration is generally for a time and under conditions sufficient to ameliorate or delay progress of clinical signs of NDD such as MS. Clinical signs will depend upon the neurodegenerative disease to be treated but may include indications of reduced cognitive, sensory or motor performance. Common clinical signs include optic neuritis, transverse myelitis, brainstem or cerebellum defects.

The agents may be administered in a convenient manner such as by the oral, intravenous (where water soluble), intraperitoneal, intramuscular, subcutaneous, intradermal, intrathecal or suppository routes or implanting (e.g. using slow release molecules). Administration may be systemic or local, although systemic is more convenient. References to systemic include intravenous, intraperitoneal, subcutaneous injection, infusion as well as administration *via* oral, rectal and nasal routes or *via* inhalation which is advantageous. Other contemplated routes of administration are by patch, cellular transfer, implant, sublingually, intraocularly, topically or transdermally. Depending upon the severity or stage of disease and integrity of the blood brain barrier, suitable antagonists are required to cross the blood brain barrier. In some embodiment, agents are administered directly to the PNS or CNS, brain stem, or cerebellum.

Pharmaceutical compositions are conveniently prepared according to conventional pharmaceutical compounding techniques. See, for example, Remington's Pharmaceutical Sciences, 18th Ed., Mack Publishing, Company, Easton, PA, U.S.A., 1990. The composition may contain the active agent or pharmaceutically acceptable salts of the active agent. These compositions may comprise, in addition to one of the active substances, a pharmaceutically acceptable excipient, carrier, buffer, stabilizer or other materials well known in the art. Such materials should be non-toxic and should not interfere with the efficacy of the active ingredient. The carrier may take a wide variety of forms depending on the form of preparation desired for administration, e.g. intravenous, oral or parenteral.

A "pharmaceutically acceptable carrier" and/or a diluent is a pharmaceutical vehicle comprised of a material that is not otherwise undesirable i.e., it is unlikely to cause a substantial adverse reaction by itself or with the active agent. Carriers may include all solvents, dispersion media, coatings, antibacterial and antifungal agents, agents for adjusting tonicity, increasing or decreasing absorption or clearance rates, buffers for maintaining pH, chelating agents, membrane or barrier crossing agents. A pharmaceutically acceptable salt is a salt that is not otherwise undesirable. The agent or composition comprising the agent may be administered in the form of pharmaceutically acceptable non-toxic salts, such as acid addition salts or metal complexes,

For oral administration, the compounds can be formulated into solid or liquid preparations such as capsules, pills, tablets, lozenges, powders, suspensions or emulsions. In preparing the compositions in oral dosage form, any of the usual pharmaceutical media may be employed, such as, for example, water, glycols, oils, alcohols, flavoring agents, preservatives, coloring agents, suspending agents, and the like in the case of oral liquid preparations (such as, for example, suspensions, elixirs and solutions); or carriers such as starches, sugars, diluents, granulating agents, lubricants, binders, disintegrating agents and the like in the case of oral solid preparations (such as, for example, powders, capsules and tablets). Because of their ease in administration, tablets and capsules represent the most advantageous oral dosage unit form, in which case solid pharmaceutical carriers are obviously employed. Tablet may contain a binder such as tragacanth, corn starch or gelatin; a disintegrating agent, such as alginic acid; and a lubricant, such as magnesium stearate. If desired, tablets may be sugar-coated or enteric-coated by standard techniques. The active agent can be encapsulated to make it stable to passage through the gastrointestinal tract. See for example, International Patent Publication No. WO 96/11698. For parenteral administration, the compound may be dissolved in a pharmaceutical carrier and administered as either a solution or a suspension. Illustrative of suitable carriers are water, saline, dextrose solutions, fructose solutions, ethanol, or oils of animal, vegetative or synthetic origin. The carrier may also contain other ingredients, for example, preservatives, suspending agents, solubilising agents, buffers and the like.

For parental administration, the antagonist may be dissolved in a carrier and administered as a solution or a suspension. When the agents are administered intrathecally, they may also be dissolved in cerebrospinal fluid. For transmucosal or transdermal (including patch) delivery, appropriate penetrants known in the art are used for delivering the antagonist. For inhalation, delivery uses any convenient system such as dry powder aerosol, liquid delivery systems, air jet nebulizers, propellant systems. For example, the formulation can be administered in the form of an aerosol or mist. The agents may also be delivered in a sustained delivery or sustained release format. For example, biodegradable microspheres or capsules or other polymer configurations capable of sustained delivery can be included in the formulation. Formulations can be modified to alter pharmacokinetics and biodistribution. For a general discussion of pharmacokinetics, see, e.g., Remington's. In some embodiments the formulations may be incorporated in lipid monolayers or bilayers such as liposomes or micelles.

Targeting therapies known in the art may be used to deliver the antagonist more specifically to certain types of cells such as neurons, oligodendrocytes, astrocytes and the like or their adjacent tissue. Targeting may help if the antagonist is unacceptably toxic, or if the dosage would otherwise be too high or inappropriate at other sites. Alternatively, targeting may assist in crossing the blood brain barrier. A number of strategies are well known in the art for improving accessibility of the nervous system to administered antagonists (Misra et a/., J Pharm Sci 6: 252-273, 2003).

In some embodiments, the antagonist is administered to neural tissue or adjacent to neural tissue. In some embodiments, administration is local to the brain, spinal cord or optic nerve.

The actual amount of active agent administered and the rate and time-course of administration will depend on the nature and severity of the disease. Prescription of treatment, e.g. decisions on dosage, timing, etc. is within the responsibility of general practitioners or specialists and typically takes into account the condition of the individual patient, the site of delivery, the method of administration and other factors known to practitioners. Examples of techniques and protocols can be found in Remington's Pharmaceutical Sciences (*supra*).

Sustained-release preparations that may be prepared are particularly convenient for long term administration of suitably stable antagonists. Examples of sustained-release preparations include semipermeable matrices of solid hydrophobic polymers containing the antagonist, which matrices are in the form of shaped articles, e.g., films, or microcapsule. Examples of sustained-release matrices include polyesters, hydrogels (for example, poly(2-hydroxyethyl-methacrylate), or poly(vinylalcohol)), polylactides, copolymers of L-glutamic acid and ethyl-L-glutamate, non-degradable ethylene-vinyl acetate, degradable lactic acid-glycolic acid copolymers, and poly-D-(-)-3-hydroxybutyric acid. While polymers such as ethylene-vinyl acetate and lactic acid-glycolic acid enable release of molecules for over 100 days, certain hydrogels release proteins for shorter time periods. Liposomes may be used which are of the small (about 200-800 Angstroms) unilamellar type in which the lipid content is greater than about 30% cholesterol, the selected proportion being adjusted for the optimal therapy.

Stabilization of antagonists may be achieved by modifying sulthydryl residues, lyophilizing from acidic solutions, controlling moisture content, using appropriate additives, and developing specific polymer matrix compositions. The *in vivo* half life of antagonists may be extended using techniques known in the art, including, for example, by the attachment of other elements such as polyethyleneglycol (PEG) groups.

The present invention further provides for combination therapy such as targeting EphA4 signaling to decrease clinical signs of a NDD such as MS, such as neural damage, and also providing an immunomodulatory or an immunosuppressive or anti-inflammatory agent or a procedure for use in ameliorating immune or inflammatory components of the disease.

The present invention further provides for combination therapy such as targeting EphA4 signaling to decrease clinical signs of MS or other NDD, such as neural damage, and also providing an immunomodulatory or an immunosuppressive or an anti-inflammatory agent or a procedure for use in ameliorating immune or inflammatory components of the disease. Immunmodulators used in the art include Beta-interferon, glatiramer acetate and natalizumab and these are contemplated for combination therapy.

Thus, the invention contemplates a method for the treatment of a NDD such as MS comprising administering an EphA4 antagonist or an EphA4 ligand antagonist together with at least one other therapeutic agent or procedure. In a similar embodiment, the invention provides an EphA4 antagonist or an EphA4 ligand antagonist together with at least one other therapeutic agent or procedure for use in treating a NDD such as MS. In another related embodiment, the invention provides an EphA4 antagonist or an EphA4 ligand antagonist for use in treating a NDD such as MS, wherein the EphA4 antagonist or EphA4 ligand antagonist is to be administered together with at least one other therapeutic agent or procedure. Similarly, the invention provides at least one therapeutic agent or procedure for use in treating a NDD such as MS, wherein the therapeutic agent or procedure is to be administered together with an EphA4 antagonist or an EphA4 ligand antagonist. In some embodiments, the antagonist reduces clinical progression of the disease. Reference to "together" includes sequential or simultaneous administration.

In some embodiments, antagonists are administered in combination with anti-inflammatory agents and other active compounds currently in use for the treatment of the target disease. Such compounds include immunomodulators such as glatiramer acetate and natalizumab, corticosteroids such as prednisolone, non-steroidal anti-inflammatory drugs, such as aspirin, ibuprofen, and COX-2 inhibitors, disease-modifying anti-rheumatic drugs, such as methotrexate, leflunomide, sulfasalazine, azathioprine, cyclosporine, hydroxychloroquine, and D-penicillamine; and biological response modifiers, such as cytokines (e.g. IFNβ) or cytokine inhibitors.

The term "nerve (neural) damage" or "axonal (neuronal) damage" includes cell death (apoptosis), cell degeneration, cell fragmentation, and reduced nerve conductance or responsiveness. In some embodiments, neuronal damage occurs in acute or chronically demyelinated lesions, however, damage may also occur in myelinated axons. In particular embodiments, markers of axonal damage permit association with clinical progression. In particular, decreased levels of axonal damage compared to controls indicates that the subject antagonists are able to protect axons from damage sufficient to reduce clinical progression in NDD such as MS.

The neurological dysfunction is a CNS or PNS dysfunction. In some embodiments, the neurological dysfunction is a dysfunction in spinal cord function, a cognitive motor or sensory function or an optic nerve function associated with clinical progress in a NDD such as MS.

In some embodiments, the neurological dysfunction is limb weakness. In some embodiments, an amount of antagonist is provided effective to reduce progressive neurological dysfunction.

In some embodiments, the antagonist preserves the function or integrity of neural tissue or reduces the rate, occurrence or amount of neural damage that takes place in a subject.

The term "damage" includes changes in the nerve (axon) that indicate loss of cellular integrity or loss of function. The term includes cell death, degeneration and fragmentation. In some embodiments the term extends to reduced nerve conductance or responsiveness or to demyelination.

In some embodiments, the antagonist preserves the function or integrity of neural tissue or reduces the rate, occurrence or amount of axon damage that takes place in a subject during the chronic progressive phases of a NDD such as MS. In some embodiments, administration is at or prior to the onset of clinical symptoms or prior to or at onset of relapse of clinical symptoms.

In some embodiments, the invention contemplates a method for treating a NDD such as MS in a subject, the method comprising:
i) screening a biological sample from the subject for axon damage; and
ii) administering an EphA4 antagonist or an EphA4 ligand antagonist under conditions sufficient to preserve function or integrity of neural tissue or reduce the rate, occurrence or amount of neural damage that takes place in the subject.

In some embodiments, is the antagonist is administered to neural tissue or adjacent to neural tissue. The neural tissue may be without limitation in the brain, CNS or spinal cord. In some embodiments, administration is local to the brain, spinal cord or optic nerve.

In some embodiments, the subject is a mammal such as a human.

In some embodiments, the antagonist binds to EphA4 and down regulates its level or activity. In an exemplary embodiment, the antagonist is an ephrin including a soluble form or a functional variant or analog thereof that binds to EphA4 and down regulates its level or activity. Soluble ephrins that are Eph receptor antagonists are well known in the art, for example as described in Gale el al., Neuron. 17(1): 9-19, 1996 and Davis *et al.,* 1994 (*supra*). These ephrins have a soluble form in that they are not membrane bound, typically because they do not include any membrane anchors and/or transmembrane domains. Other forms may be soluble in that they are biologically available at concentrations sufficient to be effective. These forms include forms that have, for example, a membrane anchor or a transmembrane domain but lack signaling capacity. These forms include micellular or proteoliposomal forms. Suitable ephrins for use in the present invention are ephrin A ephrins, such as ephrin A1, A2, A3, A4, A5, A6, or ephrin B ephrins, such as ephrin B2 or B3. In some embodiments, the ephrin is A4, A5, B2 or B3. The ephrin may be monomeric, dimeric, tetrameric or multimeric. In some embodiments, the soluble ephrin is an Fc or His fusion protein. Fc fusions are particularly contemplated.

In other embodiments, the antagonist binds to an EphA4-binding ephrin molecule and inhibits its level or activity. In an exemplary embodiment, the antagonist is a Eph receptor such as a soluble Eph receptor polypeptide or a functional variant or analog thereof. Once again, soluble Eph receptors are well known in the art, for example as described in Brantley *et al.,* 2002 (*supra*). These Eph receptors have a soluble form in that they are not membrane bound, typically because they do not include any membrane anchors and/or transmembrane domains. Other forms may be soluble in that they are biologically available at concentrations sufficient to be effective. These forms include forms that have, for example, a membrane anchor or a transmembrane domain but lack signaling capacity. These forms include micellular or proteoliposomal forms. Typically, the antagonist is a EphA4 receptor such as a soluble EphA4 receptor polypeptide or a functional variant or analog thereof. In some embodiments, the EphA4 is monomeric, dimeric, tetrameric or multimeric. In some embodiments, the EphA4 receptor is an EphA4-Fc or His fusion protein. Fc fusions are particularly contemplated.

In some embodiments, the antagonist is a protein, polypeptide or peptide such as, without limitation, a stapled peptide or a peptidomimetic. Various peptide antagonists are known in the art. These and other suitable peptide antagonists may be used in the present invention. For example, the antagonists described in Murai *et al.,* 2004 (*supra*) may be used. Methods for identifying peptide antagonists are disclosed, for example, in Koolpe et al., J Biol Chem., 280(17): 17301-17311, 2005. In other embodiments, the antagonist is a (typically small or medium sized) organic molecule such as, without limitation, a molecule derived from a library of pharmaceutically acceptable small organic molecules. Various organic molecule antagonists are known in the art. Reference may be made to Noberini et al., J Biol Chem., 283(43): 29461-29472, 2008 where small molecule antagonists are described. See also Kolb et al., Proteins, 73(1): 11-18, 2008; Chrencik et al., Structure, 14(2): 321-330, 2006; and Chrencik et al., J Biol Chem., 282(50): 36505-36513, 2007 describing methods for selecting and refining small molecule antagonists. These and other suitable organic molecule antagonists may be used in the present invention. For example, the antagonists described in Qin *et al.* 2008 (*supra*) may be used.

Isolated recombinant or synthetic polypeptides may be prepared by processes well known in the art, for example from genetically engineered host cells comprising expression systems. Practitioners are particularly directed to Sambrook *et al.,* 1989 (*supra*) and Ausubel *et al.,* 1999 (*supra*), for definitions and terms of the art and other methods known to the person skilled in the art.

The nucleotide and amino acid sequences for EphA4 and EphA4-binding ephrins are disclosed under appropriate Accession Numbers in electronic accessible databases. The appropriate nucleotide sequences may be inserted into an expression system and expressed in suitable host cells by any of a variety of well understood and routine techniques, such as is set out in Sambrook *et al.,* 1989 (*supra*) and described in the Examples. Polypeptides can be recovered and purified from recombinant cultures or from other biological sources by routine methods generally involving chromatography or filtration.

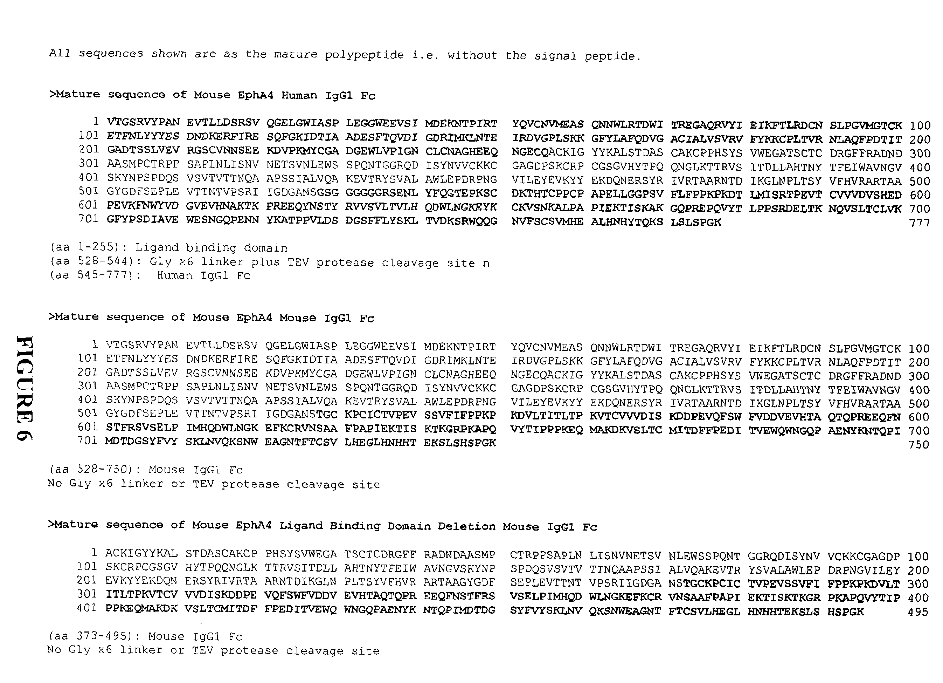

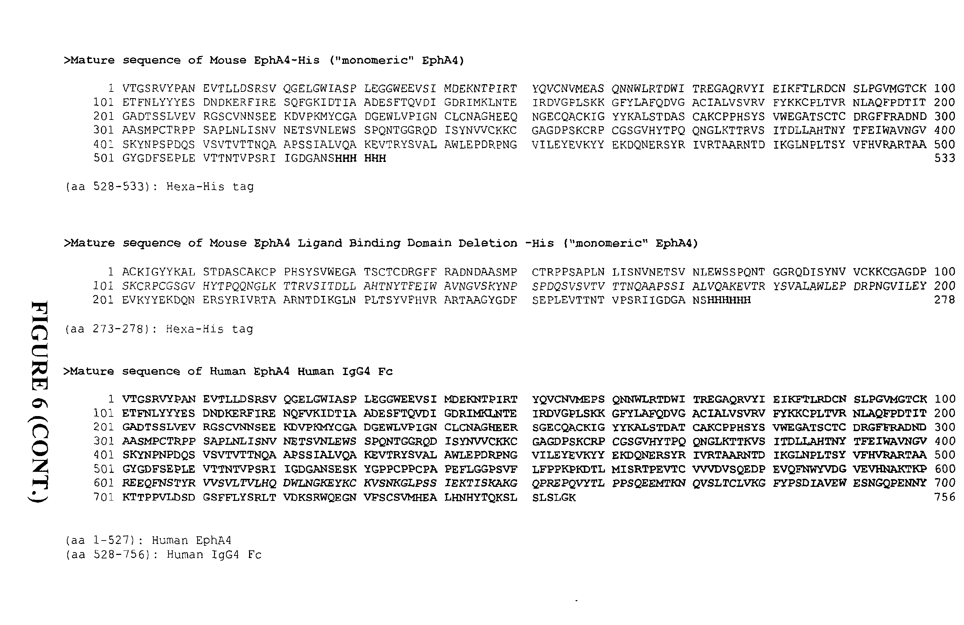

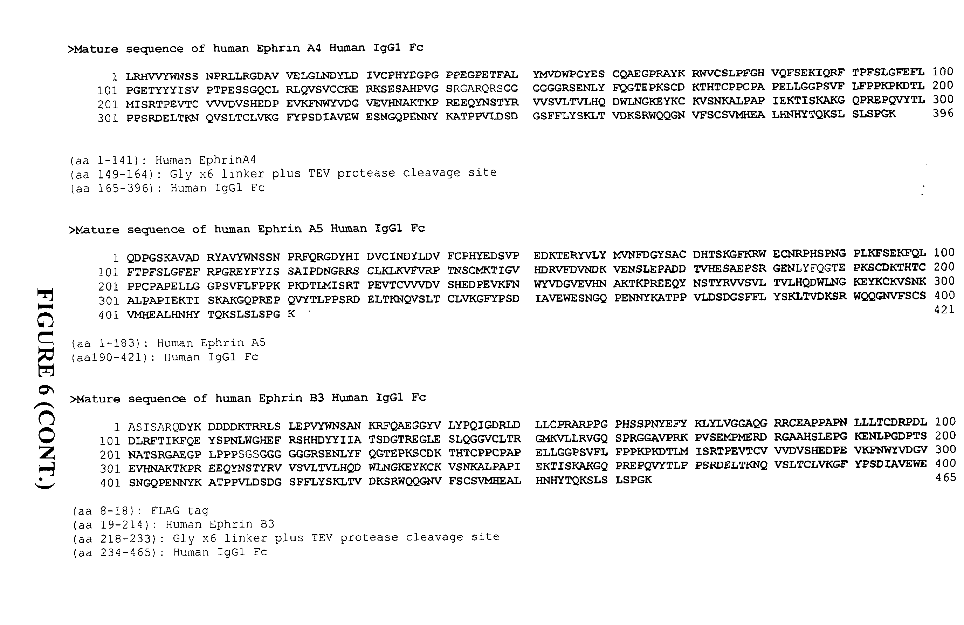

DNA encoding the EphA4 or ephrin polypeptide may be expressed recombinantly as a fusion protein with the Fc region of the human IgG heavy chain. The soluble form of ephrinA4, A5 or ephrin B3 is prepared, for example, by recombinantly expressing an ephrinA5-Fc, ephrinA3-Fc or ephrinB3-Fc fusion protein. The extracellular domain provides a soluble form of the protein, however any portion of the domain (eg. amino acids 20-546 of human EphA4) comprising the ligand binding domain (amino acids 20-274) or a functional fragment or variant is contemplated. The signal peptides may be cleaved upon expression and are not shown in Figure 6. The mouse and human homologs of EphA4 shared 98.2% amino acid sequence identity (536/546). The Fc portions in the fusion proteins facilitates protein purification using chromatography and enhances function, stability, half-life, structure or solubility of the fusion partner. Mouse Fc from IgG1 lacks complement and effector functions, however the human homolog has these functions that may interfere with effect or activity. Accordingly, the Fc may be modified or a different IgG isotype employed that does not interfere with effector cells. In an exemplary embodiment, the IgG4 isotype is employed. Ephs or ephrins may be used in monomeric, dimeric, tetrameric or multimeric forms as required.

In some embodiment, nucleic acid molecules and viruses or other vectors comprising same, if required, are used to deliver antagonists or induce silencing of an EphA4 or EphA4 ligand gene. Nucleic acids including DNA (gDNA, cDNA), RNA (sense RNAs, antisense RNAs, mRNAs, tRNAs, rRNAs, small interfering RNAs (SiRNAs), doublestranded RNAs (dsRNA), short hairpin RNAs (shRNAs), piwi-interacting RNAs (PiRNA), micro RNAs (miRNAs), small nucleolar RNAs (SnoRNAs), small nuclear (SnRNAs) ribozymes, aptamers, DNAzymes or other ribonuclease-type complexes are conveniently employed. Methods of producing chimeric or fusion constructs capable of producing DNA or RNA in eukaryotic cells are described in the art.

In some embodiments, the antagonist is an antibody or antigen-binding fragment of an antibody or aptamer that binds to EphA4 or an EphA4 ligand.

The term "antibody" as used herein encompasses a whole antibody, or a fragment thereof, for example a F(ab')2, Fab, Fab', Fv, VH or VK fragment, a single-chain antibody, a multimeric monospecific antibody or fragment thereof, or a bi- or multispecific antibody or an antigen-binding fragment thereof that exhibits the desired binding and blocking function. Reference to binding means that the fragment binds to the antigen with sufficient affinity and specificity to be useful therapeutically. Further, antibodies or their fragments are selected for the ability to bind and block target activity such as intracellular signaling. An antibody as used herein may be a polyclonal or a monoclonal antibody. The antibody may belong to any immunoglobulin class, and may be for example an IgG, for example IgG1, IgG2, IgG3, IgG4, IgE, IgM or IgA antibody. It may be of animal, for example mammalian origin, and may be for example a murine, rat or human antibody. Alternatively, the antibody may be a chimeric antibody. The term chimeric antibody is used herein to mean any antibody containing portions derived from different animal species. Particular non-limiting example is those antibodies having a variable region derived from a mouse and the antibody constant region derived from a human. Antibodies in which one or more CDR sequences are derived from a mouse and the remaining portions of the variable and the constant regions are derived from a human immunoglobulin are generally described as "humanised". An antigen binding fragment of an antibody comprises all or part or parts of the variable regions sufficient to exhibit the desired binding to EphA4 or EphA4 ligand.

Methods for preparing monoclonal and polyclonal antibodies are well known to those of ordinary skill in the art and are set forth, for example, in chapters five and six of Harlow and Lane, Antibodies, A Laboratory Manual, Cold Spring Harbor Laboratory, 1988. For example, antibodies according to the invention may be prepared by conventional immunization and recombinant DNA techniques. Thus, for example polyclonal antibodies may be obtained from the sera of animals immunised with a target protein or fragment thereof. Any suitable host, for example BALB/c mice where it is desired to obtain a mouse polyclonal antibody, may be injected with the immunogen, the serum collected and the antibody recovered therefrom. Monoclonal antibodies may be obtained from hybridomas derived from the spleen cells of an animal immunised as just discussed and fused to an appropriate "immortal" B-tumour cell. In each instance, the antibody may be recovered from either the serum or the hybridoma by making use of standard purification and or concentration techniques, for example by chromatography, using for example Protein A or by other affinity chromatography employing a protein of the invention or fragment thereof.

Once a cell line, for example a hybridoma, expressing a suitable antibody has been obtained it is possible to identify the variable region genes encoding the desired antibody, including the sequences encoding the CDRs. From here, chimeric antibodies may be obtained by preparing one or more replicable expression vectors containing at least the DNA sequence encoding the variable domain of the antibody heavy or light chain and optionally other DNA sequences encoding remaining portions of the heavy and/or light chains as desired, and transforming an appropriate cell line, e.g., a non-producing myeloma cell line, such as a mouse NS0 line, in which production of the antibody will occur. Particular methods for producing antibodies in this way are generally well known and routinely used. Antibody production includes not only the stimulation of an immune response by injection into animals, but also analogous processes such as the production of synthetic antibodies, the screening of recombinant immunoglobulin libraries for specific-binding molecules or the *in vitro* stimulation of lymphocyte populations.

Antibodies are particularly useful agents where the target has a binding site that is accessible from outside the cell. In this way, agents need not cross or fully traverse the cell membrane. Antibodies specific to an EphA4 or ephrin polypeptide can be used, for example, directly as an antagonist. The antibodies can be generated using methods that are well known in the art and include, for example, polyclonal, monoclonal, chimeric, humanized, single chain, Fab fragments, and fragments produced by a Fab expression library.

In some embodiments, the antibodies of the present invention are CDR-grafted antibodies. The term "CDR-grafted antibody" as used herein refers to an antibody molecule wherein the heavy and/or light chain contains one or more CDRs from a donor antibody (e.g., a murine monoclonal antibody) grafted into a heavy and/or light chain variable region framework of an acceptor antibody (e.g., human antibody). Construction of CDR-grafted antibodies is fully described in European Patent Application EP-A-0239400, which publication is incorporated herein by reference. Some criteria for selecting which framework residues need to be altered are described in International Patent Application WO 90/07861, incorporated herein by reference.

An antigen binding agent, or functionally active fragment thereof, which has the capacity for intracellular transmission also includes antibodies such as camelids and Ilama antibodies, scFv antibodies, intrabodies or nanobodies, e.g. scFv intrabodies and V_{HH} intrabodies. Such antigen binding agents can be made as described by Harmsen and De Haard, Appl. Microbiol. Biolechnol. 77(1): 13-22, 2007; Tibary et al., Soc. Reprod. Fertil. Suppl. 64: 297-313, 2007; Muyldermans, J. Biotechnol. 74: 277-302, 2001; and references cited therein. In one embodiment, scFv intrabodies which are able to interfere with a protein-protein interaction are used in the present invention; see for example, Visintin et al., J. Biolechnol, 135:1-15, 2008 and Visintin et al, J. Immunol. Methods, 290(1-2): 135-53, 2008 for methods for their production.

For use in the present invention, modulatory agents may comprise a cell-penetrating peptide sequence or nuclear-localizing peptide sequence such as those disclosed in Constantini et al., Cancer Biotherm. Radiopharm. 23(1): 3-24, 2008.

In some embodiments, agents that down modulate the formation, expression or activity of an EphA4 receptor may be derived from an EphA4 polypeptide or their encoding sequences or are variants or analogs thereof. Thus, for example, agents may be hydrocarbon-stapled peptides or minature proteins which are alpha-helical and cell-penetrating, and are able to disrupt protein-protein interactions (see for example, Wilder et al., ChemMedChem. 2(8): 1149-1151, 2007; and for a review, Henchey et al., Curr Opin Chem Biol. 12(6):692-697, 2008).

In some embodiments, the agents are derived from nucleic acid molecules having the published nucleotide sequences of an EphA4 or binding ligands, ephrins such as ephrin A or B ephrins; ephrinA4, A5, B2 or B3 or other EphA4 ligand gene or corrected version thereof or variants thereof. Variants include nucleic acid molecules sufficiently similar to naturally occurring forms of these molecules or their complementary forms over all or part thereof such that selective hybridisation may be achieved under conditions of medium or high stringency, or which have about 60% to 90% or 90 to 98% sequence identity to the nucleotide sequences defining a naturally occurring antagonist polypeptide sequences over a comparison window comprising at least about 15 nucleotides. Preferably the hybridisation region is about 12 to about 18 nucleobases or greater in length. Preferably, the percent identity between a particular nucleotide sequence and the reference sequence is at least about 80%, or 85%, or more preferably about 90% similar or greater, such as about 95%, 96%, 97%, 98%, 99% or greater. Percent identities between 80% and 100% are encompassed. The length of the nucleotide sequence is dependent upon its proposed function. For example, short interfering RNAs are generally about 20 to 24 nucleotides in length, whereas molecules designed to provide dominant negative functions may require full length or substantially full length molecules. As previously mentioned, homologs are encompassed. The term "homolog" or "homologs" refers broadly to functionally and structurally related molecules including those from other species. Homologs and orthologs are examples of variants.

"Hybridization" is used herein to denote the pairing of complementary nucleotide sequences to produce a DNA-DNA hybrid or a DNA-RNA hybrid. Complementary base sequences are those sequences that are related by the base-pairing rules. In DNA, A pairs with T and C pairs with G. In RNA U pairs with A and C pairs with G. In this regard, the terms "match" and "mismatch" as used herein refer to the hybridization potential of paired nucleotides in complementary nucleic acid strands. Matched nucleotides hybridize efficiently, such as the classical A-T and G-C base pair mentioned above. Mismatches are other combinations of nucleotides that do not hybridize efficiently. In the present invention, the preferred mechanism of pairing involves hydrogen bonding, which may be Watson-Crick, Hoogsteen or reversed Hoogsteen hydrogen bonding, between complementary nucleoside or nucleotide bases (nucleobases) of the strands of oligomeric compounds. For example, adenine and thymine are complementary nucleobases which pair through the formation of hydrogen bonds. Hybridization can occur under varying circumstances as known to those of skill in the art.

"Stringency" as used herein refers to the temperature and ionic strength conditions, and presence or absence of certain organic solvents, during hybridization. The higher the stringency, the higher will be the observed degree of complementarity between sequences.

"Stringent conditions" or "conditions of medium or high stringency" as used herein refers to temperature and ionic conditions under which only polynucleotides having a high proportion of complementary bases, preferably having exact complementarity, will hybridize. The stringency required is nucleotide sequence dependent and depends upon the various components present during hybridization, and is greatly changed when nucleotide analogues are used. Generally, stringent conditions are selected to be about 10° C to 20° C less than the thermal melting point (Tm) for the specific sequence at a defined ionic strength and pH. The Tm is the temperature (under defined ionic strength and pH) at which 50% of a target sequence hybridizes to a complementary probe.

It will be understood that an polynucleotide will hybridize to a target sequence under at least low stringency conditions, preferably under at least medium stringency conditions and more preferably under high stringency conditions. Reference herein to low stringency conditions include and encompass from at least about 1% v/v to at least about 15% v/v formamide and from at least about 1 M to at least about 2 M salt for hybridization at 42° C, and at least about 1 M to at least about 2 M salt for washing at 42° C. Low stringency conditions also may include 1% Bovine Serum Albumin (BSA), 1 mM EDTA, 0.5 M NaHPO4 (pH 7.2), 7% SDS for hybridization at 65° C, and (i) 2xSSC, 0.1% SDS; or (ii) 0.5% BSA, 1 mM EDTA, 40 mM NaHPO4 (pH 7.2), 5% SDS for washing at room temperature. Medium stringency conditions include and encompass from at least about 16% v/v to at least about 30% v/v formamide and from at least about 0.5 M to at least about 0.9 M salt for hybridization at 42° C, and at least about 0.5 M to at least about 0.9 M salt for washing at 42° C. Medium stringency conditions also may include 1% Bovine Serum Albumin (BSA), 1 mM EDTA, 0.5 M NaHPO4 (pH 7.2), 7% SDS for hybridization at 65° C, and (i) 2 x SSC, 0.1% SDS; or (ii) 0.5% BSA, 1 mM EDTA, 40 mM NaHPO4 (pH 7.2), 5% SDS for washing at 42° C. High stringency conditions include and encompass from at least about 31 % v/v to at least about 50% v/v formamide and from at least about 0.01 M to at least about 0.15 M salt for hybridization at 42° C, and at least about 0.01 M to at least about 0.15 M salt for washing at 42° C. High stringency conditions also may include 1% BSA, I mM EDTA, 0.5 M NaHPO4 (pH 7.2), 7% SDS for hybridization at 65° C, and (i) 0.2 x SSC, 0.1% SDS; or (ii) 0.5% BSA, 1mM EDTA, 40 mM NaHPO4 (pH 7.2), 1% SDS for washing at a temperature in excess of 65° C. Other stringent conditions are well known in the art. A skilled addressee will recognize that various factors can be manipulated to optimize the specificity of the hybridization. Optimization of the stringency of the final washes can serve to ensure a high degree of hybridization. For detailed examples, see Ausubel *et al.* (*supra*) at pages 2.10.1 to 2.10.16 and Sambrook, *et al.,* 1989 (*supra*) at sections 1.101 to 1.104.

Terms used to describe sequence relationships between two or more polynucleotides or polypeptides include "reference sequence", "comparison window", "sequence identity", "percentage of sequence identity" and "substantial identity". A "reference sequence" is at least 12 but frequently 15 to 18 and often at least 25 monomer units, inclusive of nucleotides and amino acid residues, in length. Because two polynucleotides may each comprise (1) a sequence (i.e., only a portion of the complete polynucleotide sequence) that is similar between the two polynucleotides, and (2) a sequence that is divergent between the two polynucleotides, sequence comparisons between two (or more) polynucleotides are typically performed by comparing sequences of the two polynucleotides over a "comparison window" to identify and compare local regions of sequence similarity. A "comparison window" refers to a conceptual segment of at least 6 contiguous positions, usually about 50 to about 100, more usually about 100 to about 150 in which a sequence is compared to a reference sequence of the same number of contiguous positions after the two sequences are optimally aligned. The comparison window may comprise additions or deletions (i.e., gaps) of about 20% or less as compared to the reference sequence (which does not comprise additions or deletions) for optimal alignment of the two sequences. Optimal alignment of sequences for aligning a comparison window may be conducted by computerized implementations of algorithms (GAP, BESTFIT, FASTA, and TFASTA in the Wisconsin Genetics Software Package Release 7.0, Genetics Computer Group, 575 Science Drive Madison, WI, USA) or by inspection and the best alignment (i.e., resulting in the highest percentage homology over the comparison window) generated by any of the various methods selected. Reference also may be made to the BLAST family of programs as for example disclosed by Altschul et al., Nucleic Acids Research 25:3389-3402, 1997. A detailed discussion of sequence analysis can be found in Unit 19.3 of Ausubel el al., Current Protocols in Molecular Biology, John Wiley & Sons Inc, Chapter 15, 1994-1998.

In some embodiments the present invention contemplates the use of full length polypeptides or biologically active portions or peptides such as stapled peptides of one or more of these molecules as antagonists. Biologically active portions or peptides comprise one or more binding domains that contribute to the activity of target of the antagonist, such as the ligand or receptor binding domain. In some embodiments, peptide antagonists block the G-H loop to prevent high or medium affinity binding between eph and ephrin binding domains. Other binding domains are illustrated in Figure 6. A biologically active portion or peptide such as a stapled peptide of a full length polypeptide can be a polypeptide or peptide which is, for example, 4, 5, 6, 7, 8, 9, 10, 11, 12, 13, 14, 15, 16, 17, 18, 19, 20, 21, 22, 23, 24, 25, 30, 40, 50, 60, 70, 80, 90, 100, 120, 150, 300, 350, 400, 450, 500, 550, 600 to about 640 or about 700, 800, 900, 1000, 1200, or more amino acid residues in length. Typically, ligand/receptor binding portions are approximately 150 to 300 amino acid residues. Examples are set out in Figure 6.

The amino acid sequences of illustrative antagonists are set out in SEQ ID NOs: 2 to 10 as shown in Table 1.

"Variant" antagonist polypeptides include proteins derived from the native protein such as soluble EphA4, ephrin A or ephrin B such ephrinA4, A5, B2 or B3, by deletion (truncation) or addition of one or more amino acids to the N-terminal and/or C-terminal end of the native protein; deletion or addition of one or more amino acids at one or more sites in the native protein; or substitution of one or more amino acids at one or more sites in the native protein. Variant proteins encompassed by the present invention are biologically active, that is, they continue to be inhibitors of EphA4 or ephrin mediated signaling. Variants include soluble forms lacking membrane anchors and/or transmembrane domains. Antagonist variants are selected on the basis that they inhibit or antagonise the biological activity of the EphA4 or its cell bound ligands. Such variants may result from, for example, genetic polymorphism or from human manipulation. Biologically active variants of a native polypeptide will have at least 40%, 50%, 60%, 70%, generally at least 75%, 80%, 85%, preferably about 90% to 95% or more, and more preferably about 98% or more sequence similarity with the amino acid sequence for the native protein as determined by contemporary sequence alignment programs using default parameters. A biologically active variant of an antagonist polypeptide may differ from that polypeptide generally by as much 100, 50 or 20 amino acid residues or suitably by as few as 1-15 amino acid residues, as few as 1-10, such as 6-10, as few as 5, as few as 4, 3, 2, or even 1 amino acid residue.

In one example, functionally active splice variant forms of ephrin-A4 are disclosed in Aasheim *et al., Blood, 95*(1): 221-230, 2000. The splice variant lacks 146 nucleotides at the 3' end of the open reading frame in the first part of exon IV. This results in an altered carboxy terminus and the absence of the GPI-signal sequence. COS cells transfected with the cDNA produced soluble ephrin-A4.

An EphA4 or EphA4-binding ephrin signaling antagonist polypeptide or peptide may be altered in various ways including amino acid substitutions, deletions, truncations, and insertions. Methods for such manipulations are generally known in the art. For example, amino acid sequence variants of ephrinB or ephrinA polypeptides can be prepared by introducing mutations in the encoding DNA. Methods for mutagenesis and nucleotide sequence alterations are well known in the art. See, for example, Kunkel, Proc. Natl. Acad. Sci. USA, 82: 488-492, 1985; Kunkel et al., Methods in Enzymol., 154: 367-382, 1987; United States Patent No. 4,873,192; Watson et al., Molecular Biology of the Gene, Fourth Edition, Benjamin/Cummings, Menlo Park, Calif., 1987; and the references cited therein. Guidance as to appropriate amino acid substitutions that do not affect biological activity of the protein of interest may be found in the model of Dayhoff et al., Natl. Biomed. Res. Found, 5: 345-358, 1978. Fusion proteins such as Fc fusions are particularly proposed. Such fusion proteins typically comprise linked sequences.

Methods for screening gene products of combinatorial libraries made by point mutations or truncation, and for screening cDNA libraries for gene products having a selected property are known in the art. Such methods are adaptable for rapid screening of the gene libraries generated by combinatorial mutagenesis of polypeptides. Recursive ensemble mutagenesis (REM), a technique that enhances the frequency of functional mutants in the libraries, can be used in combination with the screening assays to identify useful polypeptide variants (Arkin et al., Proc. Natl. Acad. Sci. USA, 89: 7811-7815, 1992; Delgrave et al., Protein Engineering, 6: 327-331, 1993). Conservative substitutions, such as exchanging one amino acid with another having similar properties, may be desirable.

Variant polypeptides may contain conservative amino acid substitutions at various locations along their sequence, as compared to reference amino acid sequences. A "conservative amino acid substitution" is one in which the amino acid residue is replaced with an amino acid residue having a similar side chain. Families of amino acid residues having similar side chains have been defined in the art. Amino acid residues can be further sub-classified as cyclic or noncyclic, and aromatic or nonaromatic, self-explanatory classifications with respect to the side-chain substituent groups of the residues, and as small or large. The residue is considered small if it contains a total of four carbon atoms or less, inclusive of the carboxyl carbon, provided an additional polar substituent is present; three or less if not. Small residues are, of course, always nonaromatic. Dependent on their structural properties, amino acid residues may fall in two or more classes. For the naturally-occurring protein amino acids, sub-classification according to this scheme is presented in the Table 2.

Conservative amino acid substitution also includes groupings based on side chains. Whether an amino acid change results in a functional anatagonist can readily be determined by assaying its activity in binding to its target and inhibiting EphA4 mediated signaling, receptor activation, phosphorylation and the like. Activities that can readily be assessed are known to those of skill and include assays to determine binding or dimerization or oligomerization detected by, for example, nuclear magnetic resonance spectroscopy (NMR) where heteronuclear single quantum coherence (HSQC) spectra are observed, Biacore, kinetic, affinity and pull-down analyses. Conservative substitutions are shown in Table 3 below under the heading of exemplary substitutions. More preferred substitutions are shown under the heading of preferred substitutions. Amino acid substitutions falling within the scope of the invention, are, in general, accomplished by selecting substitutions that do not differ significantly in their effect on maintaining (a) the structure of the peptide backbone in the area of the substitution, (b) the charge or hydrophobicity of the molecule at the target site, or (c) the bulk of the side chain. After the substitutions are introduced, the variants are screened for biological activity.

Antagonists may be modified by the skilled person to enhance activity, stability or, if required, ability to cross the blood brain barrier using endogenous or introduced transport molecules or carriers.

In some embodiments, analogs of ephrin or Eph polypeptides have enhanced stability and activity or reduced unfavourable pharmacological properties. They may also be designed in order to have an enhanced ability to cross biological membranes, the blood-brain barrier or to interact with only specific substrates. Thus, analogs may retain some functional attributes of the parent molecule but may posses a modified specificity or be able to perform new functions useful in the present context i.e., for administration to a subject. Analogs of polypeptide agents contemplated in this specification include but are not limited to modification to side chains, incorporating of unnatural amino acids and/or their derivatives during peptide, polypeptide or protein synthesis and the use of crosslinkers and other methods which impose conformational constraints on the proteinaceous molecule or their analogs.

Examples of incorporating unnatural amino acids and derivatives during peptide synthesis include, but are not limited to, use of norleucine, 4-amino butyric acid, 4-amino-3-hydroxy-5-phenylpentanoic acid, 6-aminohexanoic acid, t-butylglycine, norvaline, phenylglycine, ornithine, sarcosine, 4-amino-3-hydroxy-6-methylheptanoic acid, 2-thienyl alanine and/or D-isomers of amino acids. A list of unnatural amino acid contemplated herein is shown in Table 4.

Crosslinkers can be used, for example, to stabilize 3D conformations, using homo-bifunctional crosslinkers such as the bifunctional imido esters having (CH2)ₙ spacer groups with n=1 to n=6, glutaraldehyde, N-hydroxysuccinimide esters and hetero-bifunctional reagents which usually contain an amino-reactive moiety such as N-hydroxysuccinimide and another group specific-reactive moiety such as maleimido or dithio moiety (SH) or carbodiimide (COOH). In addition, peptides can be conformationally constrained by, for example, incorporation of C_{α} and N_{α}-methylamino acids and the introduction of double bonds between C_{α} and C_{β} atoms of amino acids.

In another aspect, the invention provides a use of an EphA4 antagonist or an EphA4 ligand antagonist in the manufacture of a composition for preserving function or integrity of neural tissue in a subject or for reducing the rate, occurrence or amount of neural damage in a subject. Neural tissue includes CNS and PNS tissue, brain, spinal cord, optic nerves etc. In some embodiments, the subject has or is at risk of having a condition associated with neural damage.

In a similar embodiment, methods are provided for preserving function or integrity of neural tissue or for reducing the rate, occurrence or amount of neural damage in a subject comprising administering an EphA4 antagonist or an EphA4 ligand antagonist to the subject. In some embodiments, the subject has or is at risk of having a condition associated with neural damage.

An EphA4 antagonist or an EphA4 ligand antagonist is similarly provided for use in preserving function or integrity of neural tissue or for reducing the rate, occurrence or amount of axonal damage in a subject. In some embodiments, the subject has or is at risk of having a condition associated with neural damage.

Similarly, a pharmaceutical composition is contemplated wherein the composition comprises an EphA4 antagonist or an EphA4 ligand antagonist for use in preserving function or integrity of neural tissue or for reducing the rate, occurrence or amount of axonal damage in a subject. In some embodiments, the subject has or is at risk of having a condition associated with neural damage.

Neural damage may result from a disease or disorder which results in disconnection of axons, degeneration of neurons, or demyelination. Examples of conditions that produce neural damage include traumatic lesions (e.g. caused by physical injury or surgery, and compression injuries); ischemic lesions (e.g. cerebral or spinal cord infarction and ischemia); malignant lesions; infectious lesions (e.g. resulting from an abscess or associated with infection by human immunodeficiency virus, Lyme disease, tuberculosis, syphillis, or herpes infection); degenerative lesions such as those referred to herein (e.g associated with Parkinson's disease, Alzheimer's disease, Huntington's chorea or amyotrophic lateral sclerosis); lesions associated with nutritional diseases or disorders (e.g. Vitamin B12 deficiency, folic acid deficiency, Wernicke disease, tobacco-alcohol amblyopia, Marchiafava-Bignami disease and alcoholic cerebellar degeneration); neurological lesions associated with systemic diseases (e.g. associated with diabetes, systemic lupus erythematosos, carcinoma or sarcoidosis); lesions caused by toxic substances (e.g. alcohol, lead or neurotoxin); and other demyelinated lesions (e.g. those associated with human immunodeficiency virus-associated myelopathy, transverse myelopathy of various etiologies, progressive multifocal leukoencepholopathy and central pontine myelinolysis).

A medical kit is also provided comprising a herein described antagonist together with instructions for use in the treatment of a NDD disease as described herein. A medical kit is also provided comprising an EphA4 antagonist or EphA4 ligand antagonist together with instructions for neuroprotective use in the treatment of a NDD such as MS.

The invention further provides a therapeutic protocol for treating a NDD such as MS in a subject, the protocol comprising in order, screening a biological sample from the subject for axon damage, administering an EphA4 antagonist or EphA4 ligand antagonist for a time and under conditions proposed to be sufficient to reduce further neural damage, and re-screening the subject for axon damage. In some embodiments, alternatively or in addition, the sample is tested for evidence of autoimmunity, inflammation or demyelination.

Reference to a "sample" means any biological fluid, cell, tissue, organ or portion thereof, that includes, or potentially includes, a target nucleic acid molecule or polypeptide. The term includes samples present in an individual as well as samples obtained or derived from an individual. For example, a sample can be a histologic section of a specimen obtained by biopsy, or cells that are placed in or adapted to tissue culture. A sample can be a subcellular fraction or extract, or a purified or crude nucleic acid or polypeptide preparation.

Various modes of affinity binding formats are similarly known which can be used in the diagnostic methods of the invention.. Affinity binding methods are described in common laboratory manuals such as Harlow and Lane, *Using Antibodies: A Laboratory Manual,* Cold Spring Harbor Laboratory Press, New York, 1999.

In one assay employing immunoaffinity, magnetic antibodies that bind to neural degeneration markers are used to tag the marker and a high *T*_{c} superconducting quantum interference device is used to measure the amount of free and bound antibody and hence the presence or level of the marker. A liposome immunomigration, liquid-phase competition strip immunoassay is, for example, described in Glorio-Paulet et al., J Agric Food Chem 48 (5):1678-1682, 2000.

In another embodiment, the invention provides for screening of any one or more Eph or ephrin modulators for their ability to reduce the clinical progression of an NDD such as MS disease.

In other embodiments, screens for identifying agents for treatment of a NDD such as MS are provided comprising screening agents for their ability to modulate the level or activity of a polypeptide having the functional activity of EphA4 or an EphA4 ligand.

Antagonists may be developed from natural products, combinatorial synthetic organic or inorganic compounds, peptide/polypeptide/protein, nucleic acid molecules. Libraries or phage or other display technology comprising all these are available to screen or test for suitable agents. Natural products include those from coral, soil, plant, or the ocean or Antarctic environments. Libraries of small organic molecules can be generated and screened using high-throughput technologies known to those of skill in this art. See for example United States Patent No. 5,763,623 and United States Application No. 20060167237. Combinatorial synthesis provides a very useful approach wherein a great many related compounds are synthesized having different substitutions of a common or subset of parent structures. Such compounds are usually non-oligomeric and may be similar in terms of their basic structure and function, for example, varying in chain length, ring size or number or substitutions. Virtual libraries are also contemplated and these may be constructed and compounds tested *in silico* (see for example, US Publication No. 20060040322) or by *in vitro* or *in vivo* assays known in the art. Libraries of small molecules suitable for testing are available in the art (see for example, Amezcua et al., Structure (London) 10: 1349-1361, 2002). Yeast SPLINT antibody libraries are available for testing for intrabodies which are able to disrupt protein-protein interactions (see Visintin *et al.,* (*supra*)). Examples of suitable methods for the synthesis of molecular libraries can be found in the art, for example in: DeWitt et al., Proc. Natl. Acad. Sci. USA 90: 6909, 1993; Erb et al., Proc. Natl. Acad. Sci. USA 91: 11422, 1994; Zuckermann et al., J. Med. Chem. 37: 2678, 1994; Cho et al., Science 261: 1303, 1993; Carrell et al., Angew. Chem. Int. Ed. Engl. 33: 2059, 1994; Carell et al., Angew. Chem. Int. Ed. Engl. 33: 2061, 1994; and Gallop et al., J. Med. Chem. 37: 1233, 1994.

Thus, agents can be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is suited to peptide libraries, while the other four approaches are applicable to peptide, non-peptide oligomer or small molecule libraries of compounds (Lam, Anticancer Drug Des. 12: 145, 1997; United States Patent No. 5,738,996; and United States Patent No. 5,807,683). Libraries of compounds may be presented, for example, in solution (e.g. Houghten, Bio/Techniques 13: 412-421, 1992), or on beads (Lam, Nature 354: 82-84, 1991), chips (Fodor, Nature 364: 555-556, 1993), bacteria (United States Patent No. 5,223,409), spores (United States Patent Nos. 5,571,698; 5,403,484; and 5,223,409), plasmids (Cull et al., Proc. Natl. Acad. Sci. USA 89: 1865- 1869, 1992) or phage (Scott and Smith, Science 249: 386-390, 1990; Devlin, Science 249: 404-406, 1990; Cwirla et al., Proc. Natl. Acad. Sci. USA 87: 6378-6382, 1990).

The present invention is further described by the following non-limiting Examples.

### EXAMPLES

### Example 1: Production of soluble EphA4 receptors

Recombinant mouse EphA4 mouseFc (mEphA4mFc) or human EphA4 human Fc (hEphA4hFc) fusion proteins were produced by transient transfection in mammalian cells. For cell culture, FreeStyle™ 293-F cells and the mammalian expression vector pcDNA3.1 were obtained from Invitrogen. Cells were cultured in FreeStyle™ Expression Medium (Invitrogen). All tissue culture media were supplemented with penicillin/streptomycin/fungizone reagent (Invitrogen) and cells were maintained at 37°C in incubators with an atmosphere of 8% CO₂.

Transient transfections of expression plasmids encoding mEphA4mFc or hEphA4hFc (see Figure 6) using FreeStyle™ 293-F cells was performed using 293fectin transfection reagent (Invitrogen) according to the manufacturer's instructions. Cells (1000 ml) were transfected at a final concentration of 1 x 10⁶ viable cells/ml and incubated in a Cellbag 2L (GE Healthcare) for 5 days at 37°C with an atmosphere of 8% CO₂ on a 2/10 Wave Bioreactor system 2/10 or 20/50 (GE Healthcare). The culture conditions were 35 rocks per minute with an angle of 8°. Pluronic F68 (Invitrogen), to a final concentration of 0.1 % v/v, was added 4 hours post-transfection. 24 hours post-transfection the cell cultures were supplemented with Tryptone N1 (Organotechnie, France) to a final concentration of 0.5 % v/v. The cell culture supernatants were harvested by centrifugation at 2500 rpm and were then passed through a 0.45µM filter (Nalgene) prior to purification.

cNA expression plasmids encoding the mouse EphA4 mouse Fc and human EphA4 human Fc were generated. Synthetic genes encoding mouse EphA4 fused to the mouse IgG1 Fc (mEphA4mFc), and human EphA4 fused to the human IgG4 Fc (hEphA4hFc) were constructed by GenScript Corporation (Piscataway, New Jersey) and Geneart AG (Regensburg, Germany) respectively. A Kozak sequence (GCCACC) was introduced just before the N-terminus of each protein to increase translational initiation. The codon usage of the mouse EphA4 gene, the mouse IgG1 Fc gene, the human EphA4 gene and the human IgG4 Fc gene were adapted to the codon bias of *Homo sapiens* genes. An Nhe I restriction site was introduced at the 5' end of the cDNA and an Xho I restriction site was introduced at the 3' end in order to ligate the synthetic cDNAs into Nhe I-Xho I digested pcDNA3.1. cDNA was digested with Nhe I and Xho I and ligated into pcDNA3.1. Large-scale preparations of plasmid DNA were carried out using a Qiagen Maxi or Giga Kit according to the manufacturer's instructions. The nucleotide sequences of all the plasmid constructs were verified by sequencing both strands using Big Dye Terminator v3.1 Cycle Sequencing and an Applied Biosystems Automated Sequencer.

For analysis of protein expression of mEphA4mFc or hEphA4hFc protein a 20µl aliquot of culture supernatant from a transfection of the expression construct encoding either mEphA4mFc or hEphA4hFc was electrophoresed on a 4-20% Tris-Glycine SDS polyacrylamide gel and the protein was visualised by staining with Coomassie Blue reagent. The cell culture supernatant containing either mEphA4mFc or hEphA4hFc protein was harvested by centrifugation at 2500 rpm and passed through a 0.45µM filter (Nalgene) prior to purification.

For purification, conditioned media containing EphA4 Fc was concentrated using targeted flow filtration. The concentrated media was purified by affinity chromatography using protein A. Endotoxins were removed by ion exchange chromatography. Aggregates were removed using size exclusion chromatography; i.e. to separate Fc bound dimeric EphA4 protein from other molecular weight species. Protein quantification was calculated based on 1.0 absorbance unit at 280 being equivalent to 1.3 mg/ml. Purity was based on SDS-PAGE and protein visualisation by Coomassie blue staining.

### Example 2: Assays

Disease severity, based upon histological analysis of degree of inflammatory infiltration, is based on the area of inflammatory infiltrate *per* section of spinal cord as reported (Butzkueven et al., Glia 53: 696-703, 2006). Briefly, in some embodiments, the total inflammatory lesion area of DAPI stained sections are calculated as the summed percentage area of all inflammatory infiltrates *per* total area of spinal cord in the same section. Counts are performed on 15-20 sections *per* mouse. Sections are graded using a semi-quantitative scale, (Soilu-Hanninen et al., J Neurosci Res 59: 712-721, 2000): 0, no inflammatory cells; 1, a few inflammatory cells; 2, moderate perivascular cuffing; 3, dense inflammatory cell infiltrates, parenchymal necrosis. Lesion number *per* section are quantitated.

An immunohistochemical analysis for immune cells is conducted in test subjects. Broad T cell (CD4, CD8, T cell receptor), B cell (CD19) and myeloid cell (CD11b, IBA1) markers may be investigated. If any populations are altered, then more specific subpopulation markers are employed. These markers are also used for FACS analysis of splenocytes and lymphocytes to determine whether there is a marked difference in the immunological status of the EphA4-/- mice following EAE induction or after antagonist administration.

Mature and immature oligodendrocyte numbers are quantitated in lumbar spinal cord dorsal horn white matter and in optic nerve sections. For comparison of mature oligodendrocyte density, numbers of mature oligodendrocytes are counted in sections immunostained with mouse anti-CC1 antibody (APC Ab-7, Oncogene Research Products, Germany) or from mice which have been crossed to the transgenic PLP-dsRed line, in which PLP-expressing (mature) oligodendrocytes express the marker dsRed; these mice are on C57BL/6 background. Oligodendrocyte precursors are labelled by rabbit anti-NG2 antibody (Chemicon). For analysis of oligodendrocyte apoptosis, sections are TUNEL labelled, using the TMR In Situ Cell Death Detection kit (Roche) according to the manufacturer's instructions (Butzkueven *et al.,* 2002 (*supra*)). The sections are co-stained with the CC-1 antibody. Apoptosis of TUNEL positive cells is confirmed by nuclear fragmentation or condensation using DAPI. The total number of apoptotic cells and the number of apoptotic oligodendrocytes *per* section in five, 10µm sections 100µm apart, *per* spinal cord and optic nerve *per* animal will be counted. Statistical significance is assessed by use of the Mann Whitney *U*-test. Luxol fast blue staining of sections is used to assess the extent of demyelination and remyelination over the course of the disease, as described (Butzkueven *et al.,* 2006 (*supra*)).

Axonal damage may be assessed by, for example, immunostaining for the β-amyloid precursor protein (βAPP) and hyperphosphorylation and aggregation of Tau. APP-positive axon end-bulbs and spheroids, indicative of recently severed axons, can be detected in acute MS lesions, in the active borders of chronic active lesions and to a lesser extent in chronic inactive lesions (Kornek *et al.,* 2000 (*supra*); Ferguson *et al.,* 1997 (*supra*)), while immunostaining for phosphorylated Tau reveals areas of axonal damage in MS/EAE plaques (Schneider et al. J Biol Chem. 279(53): 55833-95583, 2004). In some methods, methylene blue staining of resin-embedded sections and image analysis (Image Pro) based automated quantitation of axonal number and area in defined spinal cord regions is conveniently used. Dorsal funiculus is used as a comparable region between animals. In some embodiments, ELISA based analysis of phospho-NF in blood is an accurate marker of axonal damage. As reviewed by Gresle et al., JNeurosci Res. 86(16): 3548-3555, 2008, levels of phospho-NF-H are highly correlated with axonal loss in neuroinflammatory contexts in mice. In man, CSF pNF-H levels are potentially clinically relevant markers because levels were elvated during relapse in MS subjects. Higher levels were also associated with more rapid conversion to a secondary progressive course over a three year period, and were predictive of poor clinical outcome following relapse.

Axonal loss in optic nerve has been reported in MOG models of EAE prior to the onset of clinical symptoms (Hobom et al., Brain Pathol 14: 148-157, 2004), and tissue is examined from a 6-day time point, which is prior to the onset of clinical symptoms in the EAE model (Butzkueven *et al.,* 2002 (*supra*)). Sections from trial subjects are immunostained for phosphorylated Tau and βAPP and counterstained with H&E to highlight plaque areas. Numbers of βAPP end bulbs/spheroids are counted in plaques and in adjacent normal appearing white matter (NAWM) in longitudinal and transverse sections of lumbar spinal cord and optic nerve. Total axon density in and adjacent to plaques in spinal cord and throughout the optic nerve, are determined by automated counting of methylene blue stained cross-sections of these tissues. At least five sections of each tissue *per* animal, 50µm apart, are counted and results expressed as mean density of axons *per* mm². Statistical significance is assessed by use of the Mann Whitney *U*-test.

The number of glial fibrillary acidic protein (GFAP) positive astrocytes within plaques and in adjacent NAWM are immunostained counted and expressed as astrocytes *per* mm² at each timepoint during the course of testing. Image analysis (NIH Image J) is used to determine the amount of GFAP staining *per* field as a percentage of the total number of pixels in the field (as in Figure 3). Digital images of GFAP stained tissue will be taken at high power (x100) at multiple (5-10) sites in and around lesions, in at least 5 sections (10µm) *per* tissue, *per* mouse. To examine any differences in the level of GFAP expression, homogenates of spinal cord are processed for Western analysis. Densitometry is performed on the autoradiographs using NIH Image J software to determine relative levels of the GFAP bands and normalized to β-actin levels.

Levels of EphA4 expression are measured over the course of the disease in wildtype mice. Spinal cord and optic nerve sections are immunostained for EphA4. Levels of EphA4 expression and phosphorylation are also measured in spinal cord tissue. Lumbar spinal cord tissue are homogenised and an aliquot of lysate taken for Western analysis. The remainder are used for immunoprecipitation by anti-EphA4 antibody, then probed for phosphotyrosine and EphA4 expression.

### Example 3: Lack of EphA4 alters disease progression and functional outcome in MS models

### Induction of EAE and clinical assessment

To elucidate the role that EphA4 plays in severity and progression of EAE and to determine whether blocking EphA4 will provide a potential therapy for treatment of MS the clinical course and severity, as well as pathological and histological features of EAE in EphA4-/- (Dottori et al., Proc Natl Acad Sci U S A 95: 13248-53, 1998) and wild type mice were examined.

EphA4-/- (Dottori *et al.,* 1998 (*supra*)) mice have been backcrossed to the C57BL/6 background, therefore the MOG model of EAE is used with these mice. C57BL/6 mice are susceptible to induction of EAE using the MOG 35-55 (MEVGWRSPFSRVVHLYRNGK (SEQ ID NO: 1)) peptide (Butzkueven *et al.,* 2002 (*supra*); Slavin *et al., Autoimmunity 28*:109-20, 1998; Gold *et al.,* (*supra*)) or by passive transfer of MOG-specific T cells. The clinical course of both MOG models of EAE on the C57BL/6 background tends to be of a chronic progressive form, characterised by immune cell infiltration and focal demyelination throughout the CNS, but particularly in spinal cord and optic nerve.

The severity of the disease in EAE is evaluated daily for up to 1 month, with an additional cohort for up to 3 months using the following standard 5 point paraplegia scale: 0, no clinical symptoms; 1, fur ruffling or distal tail weakness; 1.5, tail atonia or slight hind limb paralysis; 2, complete tail paralysis; 2.5, complete tail paralysis and partial paralysis affecting one or both hindlimbs; 3, complete hindlimb paralysis; 3.5, inability to right when placed on back; 4, complete paralysis of both hind limbs and forelimb weakness or a moribund state; and 5, death. Mice that reach grade 4 are killed in accordance with ethics committee requirements and CNS tissue taken for histology. Onset of clinical symptoms using this model is typically at around 10-12 days (Butzkueven *et al.,* 2002 (*supra*))*.*

A study using n=10 EphA4-/- mice and *n*=10 control wildtype littermates was performed (see Figure 1). Onset of clinical signs commenced similarly for both cohorts but the disease was more severe in wild type mice, which reached an average clinical grade of 2.3±0.3 (maximum grade = 4; 60% maximally reached grade 2.5 or higher), while EphA4 null mice reached an average of 1.7±0.2 (maximum grade 2.75; 60% maximally reached grade 1.5 or lower). Therefore, the clinical course of EAE is not as severe in the EphA4-/- mice.

### Example 4: Immunohistological features of EAE affected tissues in EphA4-/- and wildtype mice

Analysis of spinal cord sections from the mice described in Example 1 showed a typical inflammatory infiltrate in both wildtype and EphA4 knockout mice, as assessed by DAPI (not shown) and micro-glial markers CD11b and IBA1 (not shown). There was no significant difference in the number of lesions *per* section, nor the number of DAPI nuclei in the lateral white matter.

The levels of phospho-neurofilament in blood was assessed as a surrogate marker of axonal damage that correlates well with disease severity, as well as histological features of axonal damage (see Gresle *et al,* 2008 (*supra*)). EphA4 knockout mice showed decreased levels of phospho-NF, indicating that they have had less axonal damage (see Figure 2).

### Example 5: EphA4 is expressed on astrocytes in EAE lesions

Astrocytic gliosis is a feature of MS lesions. In order to assess whether EphA4 was expressed on astrocytes in EAE lesions, spinal cord sections from C57BL/6 mice that had EAE experimentally induced by administration of the MOG35-55 peptide (Butzkueven *et al.,* 2002 (*supra*)) were immunostained for EphA4 and glial fibrillary acidic protein (GFAP) expression. EphA4 was expressed on all glial fibrillary acidic protein (GFAP) positive and therefore reactive astrocytes around lesion sites in spinal cords of mice with Grade 3 EAE (Figure 3).

### Example 6: Blockers of EphA4 following CNS damage

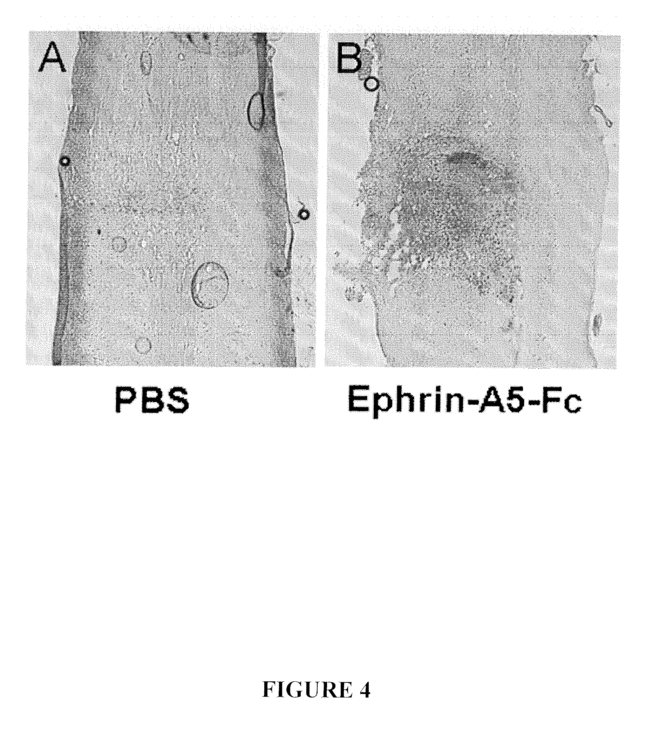

As the blood-brain-barrier is breached following CNS injury, as well as in MS and EAE lesions, this provides an access route for antagonists only at the site of CNS damage. To detect the presence of EphrinA5-Fc, sections of injured spinal cords were immunostained using biotinylated anti-human IgG, followed by the Vector ABC kit and DAB. While PBS treated spinal cords showed no labeling, EphrinA5-Fc treated spinal cords showed labeling at the lesion site, indicating that EphrinA5-Fc was able to cross the blood-brain-barrier and enter the parenchyma (Figure 4). As the blood-brain-barrier is breached in EAE lesions, treatment of EAE with IP injections of EphA4 blockers should be a viable method for delivering these molecules specifically to CNS areas affected by such lesions.

### Example 7: Addition of uncomplexed EphrinA5-Fc to astrocytes blocks IFNγ induced EphA4 activation

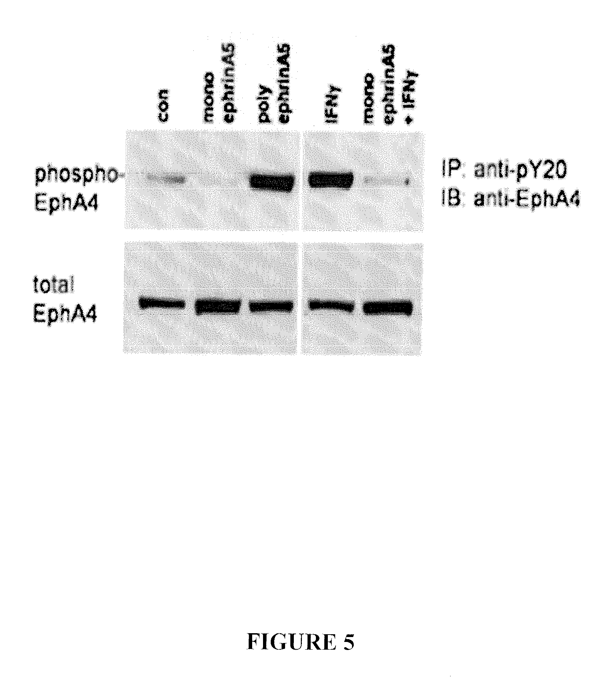

Wildtype astrocytes cultured from new born cortex were treated with ephrinA5-Fc which was either complexed with anti-human IgG Fc to generate the activating form of ephrinA5-Fc or left uncomplexed, which blocks EphA4 activation. The effect on EphA4 activation under basal conditions and in response to cytokines was assessed. Under basal conditions complexed (poly) ephrinA5-Fc increased EphA4 phosphorylation. Importantly, non-complexed (mono) EphrinA5-Fc decreased EphA4 phosphorylation under both basal conditions and in response to IFNγ (Figure 5). Therefore, in addition to regulating neuronal growth cone responses, modulation of EphA4 signaling also has direct effects on astrocytes.

### Example 8: Further studies

An EphA4 blocking agent such as soluble EphrinA5-Fc or EphA4-Fc is administered to C57BL/6 mice in which EAE has been induced by MOG as described in Example 1. Effects of the EphA4 antagonists prior to, during or after onset of clinical disease are assessed including clinical severity, inflammatory infiltrates and oligodendrocyte survival using the assays of Example 2.

In some embodiments, CHO cell transfectant lines expressing both Eph- and ephrin-human IgG1 Fc recombinant proteins are used (Coulthard el al., Growth Factors 18: 303-17, 2001). Soluble EphrinA5-Fc or EphA4-Fc protein are produced from these lines. In studies using radiolabelled ephrin A5, 65% of ephrin A5 injected IP is rapidly cleared into tissues, with similar uptake in all tissues tested. However, clearance from the blood is relatively slow, such that 5% of the injected dose remains in the circulation at 24hrs. This suggests that a daily dosing regime should be able to maintain effective circulating inhibitor. Intact, unclustered ephrin A5-Fc or EphA4-Fc are tested in EAE diseased animals.

Given preliminary data that EphrinA5-Fc, injected IP, enters the CNS parenchyma at sites of CNS damage in spinal cord injury, where the blood-brain-barrier is disrupted, initial studies in EAE use this route of administration. Penetration into the CNS parenchyma is assessed by immunostaining for human-IgG. Although soluble ephrin is expected to cross the blood-brain-barrier at the EAE lesion sites, if access is insufficient, mini-osmotic infusion pumps (Alzet) are used with a catheter inserted into the 4^{th} ventricle and the pump implanted under the skin.

Initial studies use single IP injections of ephrinA5-Fc or EphA4-Fc, at 500µg/ day/mouse, which has been found effective in spinal cord studies. Antagonists are administered from induction of disease until tissue is taken for analysis. In some embodiments, administration is prior to onset of clinical symptoms of disease or prior to induction of EAE. Subsequent studies refine the timing of commencement of treatment, such as delaying administration until onset of clinical symptoms or after assessment of axonal damage. Dose is optimised as appropriate.

The disclosure of every patent, patent application, and publication cited herein is hereby incorporated herein by reference in its entirety.

Throughout the specification the aim has been to describe the preferred embodiments of the invention without limiting the invention to any one embodiment or specific collection of features. Those of skill in the art will therefore appreciate that, in light of the instant disclosure, various modifications and changes can be made in the particular embodiments exemplified without departing from the scope of the present invention. All such modifications and changes are intended to be included within the scope of the appended claims.

**Table 1**

| ***Summary of sequence identifiers*** | |
|---|---|
| **SEQUENCE ID NO:** | **DESCRIPTION** |
| SEQ ID NO: 1 | Amino acid sequence of MOG 35-55 |
| SEQ ID NO: 2 | Mature amino acid sequence of Mouse EphA4 Human IgG1 Fc |
| SEQ ID NO: 3 | Mature amino acid sequence of Mouse EphA4 Mouse IgG1 Fc |
| SEQ ID NO: 4 | Mature amino acid sequence of Mouse EphA4 Ligand Binding Domain Deletion Mouse IgG 1 Fc |
| SEQ ID NO: 5 | Mature amino acid sequence of Mouse EphA4-His ("monomeric" EphA4) |
| SEQ ID NO: 6 | Mature amino acid sequence of Mouse EphA4 Ligand Binding Domain Delection -His ("monomeric" EphA4) |
| SEQ ID NO: 7 | Mature amino acid sequence of Human EphA4 Human IgG4 Fc |
| SEQ ID NO: 8 | Mature amino acid sequence of human Ephrin A4 Human IgG1 Fc |
| SEQ ID NO: 9 | Mature amino acid sequence of human Ephrin A5 Human IgG1 Fc |
| SEQ ID NO: 10 | Mature amino acid sequence of human Ephrin B3 Human IgG1 Fc |

**Table 2**

| ***Amino acid sub-classification*** | |
|---|---|
| **Sub-classes** | **Amino acids** |
| Acidic | Aspartic acid, Glutamic acid |
| Basic | Noncyclic: Arginine, Lysine; Cyclic: Histidine |
| Charged | Aspartic acid, Glutamic acid, Arginine, Lysine, Histidine |
| Small | Glycine, Serine, Alanine, Threonine, Proline |
| Polar/neutral | Asparagine, Histidine, Glutamine, Cysteine, Serine, Threonine |
| *Polar*/*large* | *Asparagine, Glutamine* |
| *Hydrophobic* | *Tyrosine, Valine, Isoleucine, Leucine, Methionine, Phenylalanine, Tryptophan* |
| *Aromatic* | *Tryptophan, Tyrosine, Phenylalanine* |
| *Residues that influence chain orientation* | *Glycine and Proline* |

**Table 3**

| ***Exemplary and Preferred Amino Acid Substitutions*** | | |
|---|---|---|
| **Original Residue** | **EXEMPLARY SUBSTITUTIONS** | **PREFERRED SUBSTITUTIONS** |
| Ala | Val, Leu, Ile | Val |
| Arg | Lys, Gln, Asn | Lys |
| Asn | Gln, His, Lys, Arg | Gln |
| Asp | Glu | Glu |
| Cys | Ser | Ser |
| Gln | Asn, His, Lys, | Asn |
| Glu | Asp, Lys | Asp |
| Gly | Pro | Pro |
| His | Asn, Gln, Lys, Arg | Arg |
| Ile | Leu, Val, Met, Ala, Phe, Norleu | Leu |
| Leu | Norleu, Ile, Val, Met, Ala, Phe | Ile |
| Lys | Arg, Gln, Asn | Arg |
| Met | Leu, Ile, Phe | Leu |
| Phe | Leu, Val, Ile, Ala | Leu |
| Pro | Gly | Gly |
| Ser | Thr | Thr |
| Thr | Ser | Ser |
| Trp | Tyr | Tyr |
| Tyr | Trp, Phe, Thr, Ser | Phe |
| Val | Ile, Leu, Met, Phe, Ala, Norleu | Leu |

**Table 4**

| ***Codes for non-conventional amino acids*** | | | |
|---|---|---|---|
| Non-conventional amino acid | Code | Non-conventional amino acid | Code |
| α-aminobutyric acid | Abu | L-N-methylalanine | Nmala |
| α-amino-α-methylbutyrate | Mgabu | L-N-methylarginine | Nmarg |
| aminocyclopropane- | Cpro | L-N-methylasparagine | Nmasn |
| carboxylate | | L-N-methylaspartic acid | Nmasp |
| aminoisobutyric acid | Aib | L-N-methylcysteine | Nmcys |
| aminonorbornyl- | Norb | L-N-methylglutamine | Nmgln |
| carboxylate | | L-N-methylglutamic acid | Nmglu |
| cyclohexylalanine | Chexa | L-Nmethylhistidine | Nmhis |
| cyclopentylalanine | Cpen | L-N-methylisolleucine | Nmile |
| D-alanine | Dal | L-N-methylleucine | Nmleu |
| D-arginine | Darg | L-N-methyllysine | Nmlys |
| D-aspartic acid | Dasp | L-N-methylmethionine | Nmmet |
| D-cysteine | Dcys | L-N-methylnorleucine | Nmnle |
| D-glutamine | Dgln | L-N-methylnorvaline | Nmnva |
| D-glutamic acid | Dglu | L-N-methylornithine | Nmorn |
| D-histidine | Dhis | L-N-methylphenylalanine | Nmphe |
| D-isoleucine | Dile | L-N-methylproline | Nmpro |
| D-leucine | Dleu | L-N-methylserine | Nmser |
| D-lysine | Dlys | L-N-methylthreonine | Nmthr |
| D-methionine | Dmet | L-N-methyltryptophan | Nmtrp |
| D-ornithine | Dorn | L-N-methyltyrosine | Nmtyr |
| D-phenylalanine | Dphe | L-N-methylvaline | Nmval |
| D-proline | Dpro | L-N-methylethylglycine | Nmetg |
| D-serine | Dser | L-N-methyl-t-butylglycine | Nmtbug |
| D-threonine | Dthr | L-norleucine | Nle |
| D-tryptophan | Dtrp | L-norvaline | Nva |
| D-tyrosine | Dtyr | α-methyl-aminoisobutyrate | Maib |
| D-valine | Dval | α-methyl-y-aminobutyrate | Mgabu |
| D-α-methylalanine | Dmala | α-methylcyclohexylalanine | Mchexa |
| D-α-methylarginine | Dmarg | α-methylcylcopentylalanine | Mcpen |
| D-α-methylasparagine | Dmasn | α-methyl-a-napthylalanine | Manap |
| D-α-methylaspartate | Dmasp | α-methylpenicillamine | Mpen |
| D-α-methylcysteine | Dmcys | N-(4-aminobutyl)glycine | Nglu |
| D-α-methylglutamine | Dmgln | N-(2-aminoethyl)glycine | Naeg |
| D-α-methylhistidine | Dmhis | N-(3-aminopropyl)glycine | Norn |
| D-α-methylisoleucine | Dmile | N-amino-α-methylbutyrate | Nmaabu |
| D-α-methylleucine | Dmleu | α-napthylalanine | Anap |
| D-α-methyllysine | Dmlys | N-benzylglycine | Nphe |
| D-α-methylmethionine | Dmmet | N-(2-carbamylethyl)glycine | Ngln |
| D-α-methylomithine | Dmorn | N-(carbamylmethyl)glycine | Nasn |
| D-α-methylphenylalanine | Dmphe | N-(2-carboxyethyl)glycine | Nglu |
| D-α-methylproline | Dmpro | N-(carboxymethyl)glycine | Nasp |
| D-α-methylserine | Dmser | N-cyclobutylglycine | Ncbut |
| D-α-methylthreonine | Dmthr | N-cycloheptylglycine | Nchep |
| D-α-methyltryptophan | Dmtrp | N-cyclohexylglycine | Nchex |
| D-a-methyltyrosine | Dmty | N-cyclodecylglycine | Ncdec |
| D-α-methylvaline | Dmval | N-cylcododecylglycine | Ncdod |
| D-N-methylalanine | Dnmala | N-cyclooctylglycine | Ncoct |
| D-N-methylarginine | Dnmarg | N-cyclopropylglycine | Ncpro |
| D-N-methylasparagine | Dnmasn | N-cycloundecylglycine | Ncund |
| D-N-methylaspartate | Dnmasp | N-(2,2-diphenylethyl)glycine | Nbhm |
| D-N-methylcysteine | Dnmcys | N-(3,3-diphenylpropyl)glycine | Nbhe |
| D-N-methylglutamine | Dnmgln | N-(3-guanidinopropyl)glycine | Narg |
| D-N-methylglutamate | Dnmglu | N-(1-hydroxyethyl)glycine | Nthr |
| D-N-methylhistidine | Dnmhis | N-(hydroxyethyl))glycine | Nser |
| D-N-methylisoleucine | Dnmile | N-(imidazolylethyl))glycine | Nhis |
| D-N-methylleucine | Dnmleu | N-(3-indolylyethyl)glycine | Nhtrp |
| D-N-methyllysine | Dnmlys | N-methyl-γ-aminobutyrate | Nmgabu |
| N-methylcyclohexylalanine | Nmchexa | D-N-methylmethionine | Dnmmet |
| D-N-methylornithine | Dnmorn | N-methylcyclopentylalanine | Nmcpen |
| N-methylglycine | Nala | D-N-methylphenylalanine | Dnmphe |
| N-methylaminoisobutyrate | Nmaib | D-N-methylproline | Dnmpro |
| N-(1-methylpropyl)glycine | Nile | D-N-methylserine | Dnmser |
| N-(2-methylpropyl)glycine | Nleu | D-N-methylthreonine | Dnmthr |
| D-N-methyltryptophan | Dnmtrp | N-(1-methylethyl)glycine | Nval |
| D-N-methyltyrosine | Dnmtyr | N-methyla-napthylalanine | Nmanap |
| D-N-methylvaline | Dnmval | N-methylpenicillamine | Nmpen |
| γ-aminobutyric acid | Gabu | N-(*p*-hydroxyphenyl)glycine | Nhtyr |
| L-*t*-butylglycine | Tbug | N-(thiomethyl)glycine | Ncys |
| L-ethylglycine | Etg | penicillamine | Pen |
| L-homophenylalanine | Hphe | L-α-methylalanine | Mala |
| L-α-methylarginine | Marg | L-α-methylasparagine | Masn |
| L-α-methylaspartate | Masp | L-α-methyl-t-butylglycine | Mtbug |
| L-α-methylcysteine | Mcys | L-methylethylglycine | Metg |
| L-α-methylglutamine | Mgln | L-α-methylglutamate | Mglu |
| L-α-methylhistidine | Mhis | L-α-methylhomophenylalanine | Mhphe |
| L-α-methylisoleucine | Mile | N-(2-methylthioethyl)glycine | Nmet |
| L-α-methylleucine | Mleu | L-α-methyllysine | Mlys |
| L-α-methylmethionine | Mmet | L-α-methylnorleucine | Mnle |
| L-α-methylnorvaline | Mnva | L-α-methylornithine | Morn |
| L-α-methylphenylalanine | Mphe | L-α-methylproline | Mpro |
| L-α-methylserine | Mser | L-α-methylthreonine | Mthr |
| L-α-methyltryptophan | Mtrp | L-α-methyltyrosine | Mtyr |
| L-α-methylvaline | Mval | L-N-methylhomophenylalanine | Nmhphe |
| N-(N-(2,2-diphenylethyl) | Nnbhm | N-(N-(3,3-diphenylpropyl) | Nnbhe |
| carbamylmethyl)glycine | carbamylmethyl)glycine | | |
| 1-carboxy-1-(2,2-diphenyl-ethylamino)cyclopropane | Nmbc | | |

**TABLE 5**

| ***List of abbreviations*** | |
|---|---|
| **ABBREVIATION** | |
| EAE | experimental autoimmune encephalomyelitis |
| ELISA | enzyme-linked immunosorbent assay |
| GFAP | Glial fibrillary acidic protein |
| NDD | neurodegenerative diseases |
| PBS | phosphate buffered saline |
| MOG | myelin oligodendrocyte glycoprotein |
| CNS | central nervous system |
| PNS | peripheral nervous system |
| RNA | ribonucleic acid |
| PDZ | post synaptic density protein (PSD95), Drosophila disc large tumor suppressor (DlgA), and zonula occludens-1 protein (zo-1) |
| SAM | sterile alpha motif |
| GPI | glycophosphatidyl inositol |
| MS | multiple sclerosis |
| Eph | erythropoietin producing hepatocellular |
| LIF | leukocyte inhibitory factor |
| IFNγ | interferon gamma |
| Fc | fragment crystalisable region |
| IFNβ | interferon Beta |
| DAPI | 4',6'-diamino-2-phenylindole |

### BIBLIOGRAPHY

Aasheim et al., Blood, 95(1): 221-230, 2000
Altschul et al., Nucleic Acids Research 25:3389-3402, 1997
Amezcua et al., Structure (London) 10: 1349-1361, 2002
Arkin et al., Proc. Natl. Acad. Sci. USA, 89: 7811-7815, 1992
Ausubel et al., Current Protocols in Molecular Biology, Supplement 47, John Wiley & Sons, New York, 1999
Ausubel et al., Current Protocols in Molecular Biology, John Wiley & Sons Inc, Chapter 15, 1994-1998
Barnes et al., Brain 114: 1271-80, 1991
Bjartmar et al., JNeurol Sci 206: 165-71, 2003
Bjartmar et al., Neurology 57:1248-52, 2001
Brantley et al., Oncogene 21: 7011-26, 2002
Butzkueven et al. Nat Med 8: 613-9, 2002
Butzkueven et al., Glia 53: 696-703, 2006
Carell et al., Angew. Chem. Int. Ed Engl. 33: 2061, 1994
Carrell et al., Angew. Chem. Int. Ed. Engl. 33: 2059, 1994
Cheng et al., Cell 82: 371-81, 1995
Cho et al., Science 261: 1303, 1993
Chrencik et al., J Biol Chem., 282(50): 36505-36513, 2007
Chrencik et al., Structure, 14(2): 321-330, 2006
Coligan et al., Current Protocols In Protein Science, John Wiley & Sons, Inc., 1995-1997
Constantini el al., Cancer Biotherm. Radiopharm. 23(1): 3-24, 2008
Coulthard el al., Growth Factors 18: 303-17, 2001
Cull et al., Proc. Natl. Acad. Sci. USA 89: 1865- 1869, 1992
Cwirla et al., Proc. Natl. Acad. Sci. USA 87: 6378-6382, 1990
Davis el al., Science 266: 816-9, 1994
Dayhoff et al., Natl. Biomed. Res. Found, 5: 345-358, 1978
De Stefano et al., Brain 121 (Pt 8): 1469-77, 1998
Delgrave et al., Protein Engineering. 6: 327-331, 1993
Devlin, Science 249: 404-406, 1990
DeWitt et al., Proc. Natl. Acad Sci. USA 90: 6909, 1993
Dobrzanski et al., Cancer Res 64: 910-9, 2004
Dottori et al., Proc Natl Acad Sci USA 95: 13248-53, 1998
Erb et al., Proc. Natl. Acad. Sci. USA 91: 11422, 1994
Ferguson et al., Brain 120: 393-9, 1997
Fodor, Nature 364: 555-556, 1993
Fu et al., Nature Neuroscience 10: 67 - 76, 2007
Gale et al., Neuron. 17(1): 9-19, 1996
Gallop et al., J Med Chem. 37: 1233, 1994
Gold et al., Brain. 129(Pt 8): 1953-1971, 2006
Goldshmit et al., Brain Res Brain Res Rev 52: 327-45, 2006
Goldshmit et al., JNeurosci 24: 10064-73, 2004
Gonen et al., Neurology 54: 15-9, 2000
Gresle et al., JNeurosci Res. 86(16): 3548-3555, 2008
Harlow and Lane, Antibodies, A Laboratory Manual, Cold Spring Harbor Laboratory, 1988
Harlow and Lane, Using Antibodies: A Laboratory Manual, Cold Spring Harbor Laboratory Press, New York, 1999
Harmsen and De Haard, Appl. Microbiol. Biotechnol. 77(1): 13-22, 2007
Henchey et al., Curr Opin Chem Biol. 12(6):692-697, 2008
Henkemeyer el al., Cell 86: 35-46, 1996
Hobom el al., Brain Pathol 14: 148-157, 2004
Horner and Gage, Nature. 407(6807): 963-970, 2000
Houghten, Bio/Techniques 13: 412-421, 1992
Kidd el al., Brain 122 17-26, 1999
Kolb et al., Proteins, 73(1): 11-18, 2008
Koolpe el al., J Biol Chem., 280(17): 17301-17311, 2005
Kornek et al., Am J Pathol 157: 267-76, 2000
Kunkel et al., Methods in Enzymol., 154: 367-382, 1987
Kunkel, Proc. Natl. Acad Sci. USA, 82: 488-492, 1985
Lam, Anticancer Drug Des. 12: 145, 1997
Lam, Nature 354: 82-84, 1991
Liedtke et al., Am J Pathol 152: 251-9, 1998
Lo et al., J Neurophysiol 90: 3566-71, 2003
Misra et al., J Pharm Sci 6: 252-273, 2003
Monschau et al., Embo J 16: 1258-67, 1997
Murai et al., Mol Cell Neurosci. 24(4): 1000-1011, 2004
Muyldermans, J Biotechnol. 74: 277-302, 2001
Noberini et al., J Biol Chem., 283(43): 29461-29472, 2008
Ohta et al., Mech Dev 64: 127-35, 1997
Pasquale, Nat Rev Mol Cell Biol. 6(6): 462-75, 2005
Peterson et al., Ann Neurol 50: 389-400, 2001
PubChem Bioassay AID No. 689 (*Colorimetric assay for HTS discovery of chemical*
*inhibitors of EphA4 receptor antagonists*)
Qin et al., J Biol Chem. 283(43): 29473-29484, 2008
Raine et al., Lab Invest 31: 369-80, 1974
Remington's Pharmaceutical Sciences, 18th Ed., Mack Publishing, Company, Easton, PA, U.S.A., 1990
Sambrook el al., Molecular Cloning: A Laboratory Manual (2nd ed.). Cold Spring Harbour Laboratory, Cold Spring Harbour, NY, 1989
Schneider et al. J Biol Chem. 279(53): 55833-95583, 2004
Scott and Smith, Science 249: 386-390, 1990
Shamah el al., Cell 105: 233-44, 2001
Smith et al.. Brain Res 264: 241-53, 1983
Sobel, Brain Pathol. 15(1): 35-45, 2005
Soilu-Hanninen el al., J Neurosci Res 59: 712-721, 2000
Stein et al., Genes Dev 12: 667-78, 1998
Tibary et al., Soc. Reprod. Fertil. Suppl. 64: 297-313, 2007
Trapp al., Curr Opin Neurol 12: 295-302, 1999
Trapp et al., N Engl J Med 338: 278-85, 1998
Visintin et al, J. Immunol. Methods, 290(1-2): 135-53, 2008
Visintin et al., J Biotechnol, 135:1-15, 2008
Wahl et al., J Cell Biol 149: 263-70, 2000
Watson et al., Molecular Biology of the Gene, Fourth Edition, Benjamin/Cummings, Menlo Park, Calif., 1987
Wilder et al., ChemMedChem. 2(8): 1149-1151, 2007
Wilkinson, Nat Rev Neurosci. 2(3): 155-164, 2001
Wu et al., Neuroimage 37: 1138-47, 2007
Wujek et al., JNeuropathol Exp Neurol 61: 23-32, 2002
Zuckermann et al., J. Med Chem. 37: 2678, 1994

## Claims

1. A method for treating a neurodegenerative disease (NDD) in a subject in need thereof, the method comprising administering to the subject an EphA4 antagonist or an EphA4 ligand antagonist.

2. The method of claim 1 wherein the antagonist binds to EphA4 and down regulates its level or activity.

3. The method of claim 2 wherein the antagonist is:
a) a soluble ephrin or a functional variant or an analog thereof, preferably a soluble ephrin A5, soluble ephrin B2 or soluble ephrin B3; or
b) an antibody or antigen binding fragment that binds to EphA4 and down regulates its level or activity.

4. The method of claim 2 wherein the antagonist binds to an EphA4-binding ephrin and down regulates its level or activity.

5. The method of claim 4 wherein the antagonist is:
a) a soluble EphA4 or a functional variant or an analog thereof, preferably wherein the soluble EphA4 is EphA4-Fc; or
b) an antibody or an antigen binding fragment that binds to an EphA4-binding ephrin and down regulates its level or activity.

6. The method of claim 1 wherein the antagonist is a nucleic acid, polypeptide, peptide or organic molecule that binds to EphA4 or an EphA4-ligand or a nucleic acid encoding same and down regulates its level or activity.

7. The method of any one of claims 1 to 6 wherein the antagonist is administered together with at least one other therapeutic agent or procedure.

8. The method of any one of claims 1 to 7, wherein the antagonist reduces clinical progression of the disease.

9. The method of any one of claims 1 to 8 wherein the subject is at risk of a NDD or exhibits clinical signs of a NDD.

10. The method of any one of claims 1 to 9 wherein the neurodegenerative disease is multiple sclerosis (MS) or a variant of MS.

11. The method of any one of claims 1 to 10 wherein the method comprises screening a biological sample from the subject for axon damage.

12. The method of any one of claims 1 to 11 wherein the subject exhibits or is likely to exhibit less nerve damage or less progressive neurological dysfunction.

13. The method of claim 12 wherein the antagonist preserves the function or integrity of neural tissue or reduces the rate, occurrence or amount of neural damage that takes place in the subject, preferably wherein the antagonist preserves the function or integrity of neural tissue or reduces the rate, occurrence or amount of neural damage that takes place in the subject during the chronic progressive phases of a NDD such as MS.

14. The method of claim 12 wherein the neurological dysfunction is limb weakness.

15. The method of any one of claims 1 to 14 wherein administration is prior to or at onset of one or more clinical symptoms or prior to or at onset of relapse of one or more clinical symptoms of NDD and/or administration is local to the brain, spinal cord, or optic nerve.
